# EUROPEAN PATENT SPECIFICATION

(11) **EP 3 890 886 B1**
(45) Date of publication and mention of the grant of the patent: **29.10.2025**
(21) Application number: 19828438.2
(22) Date of filing: 04.12.2019
(51) Int. Cl.: B01L 3/00, C12Q 1/6858, F16K 7/00, B01L 7/00

(54) **CONTROLLING DNA CONCENTRATION FOR STR ANALYSIS**
DNA KONZENTRATIONS KONTROLLE ZUR STR ANALYSIS
CONTROL DE LA CONCENTRATION DE L'ADN POUR L'ANALYSE DE STR

(30) Priority: 04.12.2018 US 201862775306 P
(43) Date of publication of application: 13.10.2021
(73) Proprietor: Integenx, Inc., Pleasanton, CA 94588 (US)
(72) Inventor: VANGBO, Mattias, Pleasanton, California 94588 (US); KING, David, Menlo Park, California 94025 (US)
(74) Representative: Thermo Fisher Scientific- Life Sciences Solutions Group
(86) International application number: PCT/US2019/064525
(87) International publication number: WO 2020/117969

(56) References cited:
- WO-A1-2010/041214
- WO-A1-2016/205428
- GB-A- 2 516 672
- FANG R ET AL: "The HID EVOlution System for Automation of DNA Quantification and Short Tandem Repeat Analysis", JOURNAL OF THE ASSOCIATION FOR LABORATORY AUTOMATION, ELSEVIER, vol. 15, no. 1, 1 February 2010 (2010-02-01), pages 65 - 73, XP026817835, ISSN: 1535-5535, [retrieved on 20091225]
- STEVAN JOVANOVICH ET AL: "Developmental validation of a fully integrated sample-to-profile rapid human identification system for processing single-source reference buccal samples", FORENSIC SCIENCE INTERNATIONAL: GENETICS, vol. 16, 1 May 2015 (2015-05-01), Netherlands, pages 181 - 194, XP055283244, ISSN: 1872-4973, DOI: 10.1016/j.fsigen.2014.12.004
- JACKLYN BUSCAINO ET AL: "Evaluation of a rapid DNA process with the RapidHIT ID system using a specialized cartridge for extracted and quantified human DNA", FORENSIC SCIENCE INTERNATIONAL: GENETICS, vol. 34, 1 May 2018 (2018-05-01), Netherlands, pages 116 - 127, XP055672052, ISSN: 1872-4973, DOI: 10.1016/j.fsigen.2018.02.010

## Description

### BACKGROUND

Polymerase Chain Reaction ("PCR") is a technique used to create duplicates of selected sequences of DNA. In theory each time the process is repeated (a PCR "cycle")-subjecting a mixture of DNA-containing sample and reagents to a series of temperature ramps and dwell times-the segments of DNA having the selected sequences are copied and their concentration doubled. However, due to various sources of imperfection, not every segment of the DNA sequences is successfully duplicated in each cycle, an effect that can be expressed as amplification efficiency. Commonly the efficiency of a well-designed PCR reaction can be over 95% or 98% or 99%.

Often multiple samples to be amplified by PCR are processed concurrently. Current standard PCR protocols (including PCR protocols for amplifying Short Tandem Repeats ("STRs")) implemented in microtiter plates will, as designed, subject all samples being processed in the plate to the same number of amplification cycles. Also, a single sample may be split and subjected to PCR conditions in separate thermal cyclers as disclosed e.g. at WO2016205428, which describes a microfluidic device for multi-channel sequencing characterised in that the device is a cartridge that has a plurality of reaction chambers each having a controlled thermal cycler. The sample is transferred from the input port to different reaction chambers in order to split the sample. A similar device is disclosed at GB2516672. WO2010041214 discloses a fluidic device comprising a plurality of chambers suitable for performing PCR. Each chamber has a separated heater and temperature sensors such that different PCR conditions can be applied..For the amplified output to meet specified quality requirements, the input concentration of DNA in the different samples must be normalized to one another and as an ensemble to a process window specified by the optimized protocol. Common protocol uses 25 to 31 cycles of amplification depending on the sample/assay type and the expected range of concentrations.

A typical protocol may involve first purifying or up-concentrating sample DNA; followed by DNA quantification, commonly using quantitative PCR ("qPCR"); and finally diluting the purified and quantified sample by mixing metered amounts of liquids to achieve a target concentration for the protocol.

Metering and mixing are challenging steps in small volume fluidics, particularly mesofluidics and microfluidics, in particular when the volumes of the constituents are not pre-determined. Also, for an accurate quantification, qPCR requires sophisticated instrumentation. This is partly because the error introduced by the amplification efficiency discussed above can only be estimated. Only complex instruments, having very low detection limits for amplified product, can provide high accuracy estimates of initial concentration.

Certain applications such as high-throughput, low-cost forensic STR analysis cannot justify such sophisticated instrumentation. Thus, they rely on rough estimates of sample concentration such as "high" and "low." But this is not appropriate for handling relatively arbitrary sets of samples. Furthermore, for crime scene applications, current US FBI regulations require quantifying sample DNA concentration and, if necessary, sample normalization. Fang R et al: disclosed in "The HID EVOlution System for Automation of DNA Quantification and Short Tandem Repeat Analysis", Journal Of The Association For Laboratory Automation, ELSEVIER, Vol. 15, No. 1, pages 65-73, the importance of quantifying the DNA present in the sample in order to normalise it and perform a STR PCR on a diluted sample in order for the results to be useful in forensic.

### SUMMARY

Disclosed herein are methods and systems of DNA sample preparation for an assay using an amplification assay, for example, quantitative PCR ("qPCR") and Short Tandem Repeat ("STR") PCR. qPCR may be used to determine information about DNA concentration in a sample. STR PCR may be used to amplify the DNA sample for use with an assay.

In one aspect of the embodiments herein, a method of preparing a DNA-containing sample for an assay is provided.

In a first embodiment, the method including: receiving an input sample comprising DNA; directing a first portion of DNA from the input sample to a first amplification chamber (e.g., a first PCR chamber) of a first cartridge; directing a second portion of DNA from the input sample to a second amplification chamber (e.g., a second PCR chamber) of the first cartridge or of a second cartridge; initiating an amplification assay (e.g., a qPCR assay) on the first portion in the first PCR chamber; before completing the qPCR assay in the first PCR chamber, initiating STR PCR on the second portion in the second PCR chamber; detecting an output signal from the first PCR chamber, which output signal provides information about DNA concentration in the first portion of DNA from the input sample; and completing the STR PCR by conducting a predetermined number of amplification cycles on the second portion in the second PCR chamber, wherein the predetermined number of amplification cycles in based on the output signal from the first PCR chamber.

In another embodiment, the method including: receiving an input sample comprising DNA into a cartridge; directing a first portion of DNA from the input sample to a first PCR chamber of the cartridge; directing a second portion of DNA from the input sample to a second PCR chamber of the cartridge; initiating a qPCR assay on the first portion in the first PCR chamber; before completing the qPCR assay in the first PCR chamber, initiating STR PCR on the second portion in the second PCR chamber; detecting an output signal from the first PCR chamber, which output signal provides information about DNA concentration in the first portion of DNA from the input sample and completing the STR PCR by conducting a predetermined number of amplification cycles on the second portion in the second PCR chamber, wherein the predetermined number of amplification cycles in based on the output signal from the first PCR chamber.

In various implementations, the input sample includes DNA obtained at a crime scene. Examples of sources of DNA obtained at a crime include bones, teeth, saliva, hair, blood, semen, skin, and any combination thereof.

In certain embodiments, the cartridge is a unitary structure with a plurality of fluid channels and a plurality of fluid chambers. In some embodiments, the cartridge can be or comprise a plurality of (separate and/or distinct) cartridges. The plurality of cartridges can each have a plurality of fluid channels and a plurality of fluid chambers. Alternatively, each of the plurality of cartridges can include one or more of the plurality of fluid channels and the plurality of fluid chambers.

Throughout the present application, reference may be made to "a cartridge" or "the cartridge." It will be appreciated that each of the various aspects, embodiments, or implementations of the present application can alternatively include a plurality of cartridges, each having one or more of the plurality of fluid channels and the plurality of fluid chambers.

In some implementations, the input sample is about 1 picogram to 100 nanograms of DNA. In other implementations, the DNA from the input sample is not diluted by adding liquid to the first or second portions of DNA from the input sample prior to initiating qPCR or STR PCR.

In some implementations, the assay is capillary electrophoresis of STR alleles. In various implementations, the qPCR assay is performed in a manner meeting the requirements of the US Quality Assurance Standard for DNA Databasing Laboratories, September 2011.

In some embodiments, the method further includes, before directing the first portion of DNA from the input sample to a first PCR chamber, dividing the DNA from the input sample into the first portion and the second portion DNA, and wherein the first portion of DNA from the input sample has a volume within about twenty percent of the volume of the second portion of DNA from the input sample. In various embodiments, the method further includes extracting DNA from the input sample prior to initiating qPCR on the first portion of DNA from the input sample. In some implementations, extracting the DNA includes contacting the input sample with DNA capture beads. In further implementations, the second PCR chamber includes an absorbent material that holds the second portion of DNA from the input sample during STR PCR.

In various embodiments, the qPCR and STR PCR are performed using a single thermocycler, and during at least a portion of the qPCR performed at the same time as at least a portion of the STR PCR, the qPCR and the STR PCR are subject to the same thermal profile. In further embodiments, the qPCR and the STR PCR are performed using separate thermocyclers.

In another aspect of the embodiments herein, a system for preparing a sample for an assay is provided, the system including: (a) an interface for receiving and operating on a cartridge; (b) logic for causing the following operations to be performed in the cartridge: receiving an input sample comprising DNA; directing a first portion of DNA from the input sample to a first PCR chamber of the cartridge; directing a second portion of DNA from the input sample to a second PCR chamber of the cartridge; initiating a qPCR on the first portion of DNA from the input sample in the first PCR chamber; before completing the qPCR in the first PCR chamber, initiating STR PCR on the second portion of DNA from the input sample in the second PCR chamber; detecting an output signal from the first PCR chamber, which output signal provides information about DNA concentration in the first portion of DNA from the input sample; and completing the STR PCR by conducting a predetermined number of amplification cycles on the second portion in the second PCR chamber, wherein the predetermined number of amplification cycles is based on the output signal from the first PCR chamber.

In another aspect of the embodiments herein, a system for preparing a sample for an assay is provided, the system including: (a) a plurality of interfaces for receiving and operating a respective plurality of cartridges; (b) logic for causing the following operations to be performed in the plurality of cartridges: receiving an input sample comprising DNA; directing a first portion of DNA from the input sample to a first PCR chamber of a first cartridge; directing a second portion of DNA from the input sample to a second PCR chamber of a second cartridge; initiating a qPCR on the first portion of DNA from the input sample in the first PCR chamber; before completing the qPCR in the first PCR chamber, initiating STR PCR on the second portion of DNA from the input sample in the second PCR chamber; detecting an output signal from the first PCR chamber, which output signal provides information about DNA concentration in the first portion of DNA from the input sample; and completing the STR PCR by conducting a predetermined number of amplification cycles on the second portion in the second PCR chamber, wherein the predetermined number of amplification cycles is based on the output signal from the first PCR chamber.

In various embodiments, the system includes one or more actuators for dynamically controlling movement of fluid in fluidic passages of the cartridge. In various embodiments, the system includes a thermocycler configured to cause the qPCR in the first PCR chamber. In further embodiments, the thermocycler is further configured to cause the STR PCR in the second PCR chamber.

In some embodiments, the system includes at least one thermocycler configured to (i) cause the qPCR in the first PCR chamber and (ii) cause the STR PCR in the second PCR chamber. In various implementations, the system includes (a) a capillary containing a separation medium and having inlet and distal ends; (b) an inlet in communication with the capillary inlet and configured to receive samples containing DNA fragments having a plurality of different sizes; (c) an interrogation region configured to detect DNA fragments moving through the capillary; and (d) a power source configured to apply voltage between inlet and distal ends of the capillary. In further implementations, the system includes a chassis enclosing the interface(s) and at least some of the logic.

In various embodiments, the cartridge is a unitary structure including a plurality of fluid channels and a plurality of fluid chambers. In some embodiments, the logic is configured to cause the recited operations in a manner such that neither the input sample nor the second portion of DNA from the input sample is quantitated prior to initiating the STR PCR. In further embodiments, the logic is configured to cause the recited operations in a manner such that, after starting the qPCR but before completing the qPCR on the first portion of DNA from the input sample in the first PCR chamber, STR PCER is initiated by providing one or more PCR reagents to the second PCR chamber and/or directing the second portion of DNA from the input sample to the second PCR chamber.

In some implementations, the logic is configured to cause the recited operations in a manner such that initiating the STR PCR begins between five and fifteen PCR cycles after starting the qPCR. In various implementations, the logic is configured to cause the recited operations in a manner such that initiating the STR PCR includes providing the one or more PCR reagents to the second PCR chamber. In further implementations, the logic is configured to cause the recited operations in a manner such that directing the first portion of DNA from the input sample to the first PCR chamber and directing the second portion of DNA from the input sample to the second PCR chamber occur at substantially the same time.

In various embodiments the assay is capillary electrophoresis of STR alleles. In some embodiments, the logic is configured to cause the recited operations in a manner such that the qPCR is performed in a manner meeting the requirements of the US Quality Assurance Standard for DNA Databasing Laboratories, September 2011. In some embodiments the logic is further configured to cause extracting DNA from the input sample prior to initiating qPCR on the first portion of DNA from the input sample.

In another aspect of the embodiments herein, a system for conducting a PCR assay is provided, including: an input port for receiving a sample containing a target nucleic acid; a first PCR chamber configured to receive a first portion of the sample; a second PCR chamber configured to receive a second portion of the sample; and one or more channels configured to transfer the sample from the input port and to subsequently split the sample between the first PCR chamber and the second PCR chamber.

In some embodiments, the system is a cartridge configured for use in a processing system. In various embodiments, the first PCR chamber is configured to provide a qPCR assay and the second chamber is configured to provide STR PCR. In further embodiments, the system includes an input chamber including the input port, the chamber optionally including an inlet port configured to introduce a lysis solution into the input chamber.

In some embodiments, the system includes at least one of: a sample preparation chamber disposed between the input port and the PCR chambers along a channel of the one or more channels; a first reagent port configured to deliver a first reagent mixture to the first PCR chamber and a second reagent port configured to deliver a second reagent mixture to the second PCR chamber, the second reagent mixture being chemically different from the first reagent mixture; a waste chamber configured to collect excess of the sample and/or waste produced during clean-up of the sample; a mix chamber configured to receive the second portion after an STR PCR assay is performed in the second chamber and further process with one or more components; or an outlet port fluidly coupled to the mix chamber.

In another aspect of the embodiments herein, a system for conducting a PCR assay is provided, including: a cartridge including: an input port for receiving a sample containing a target nucleic acid; a first PCR chamber configured to receive a first portion of the sample; a second PCR chamber configured to receive a second portion of the sample; and one or more channels configured to transfer the sample from the input port and to subsequently split the sample between the first PCR chamber and the second PCR chamber; and a processing system configured to receive the cartridge, the processing system including: a first thermal cycler that is in physical and thermal contact with the first PCR chamber during use; and a second thermal cycler that is in physical and thermal contact with the second PCR chamber during use.

In some embodiments, the system includes a thermal controller including a memory including instructions to operate the first thermal cycler and the second thermal cycler independently of one another. In various embodiments the system includes one or more detectors configured to detect fluorescent signal produced during a PCR assay in the first PCR chamber. In further embodiments the first thermal cycler is configured to perform a qPCR on the first portion and the second thermal cycler is configured to perform a STR PCR on the second portion.

In various implementations, the cartridge further includes an input chamber including the input port, the chamber optionally including an inlet port configured to introduce a lysis solution into the input chamber. In some implementations, the cartridge further includes at least one of: a sample preparation chamber disposed between the input port and the PCR chambers along a channel of the one or more channels; a first reagent port configured to deliver a first reagent mixture to the first PCR chamber and a second reagent port configured to deliver a second reagent mixture to the second PCR chamber, the second reagent mixture being chemically different from first reagent mixture; a waste chamber configured to collect excess of the sample and/or waste produced during clean-up of the sample; a mix chamber configured to receive the second portion after an STR PCR assay is performed in the second chamber and further process with one or more components; or an outlet port fluidly coupled to the mix chamber.

In another aspect of the embodiments herein, a system for preparing a sample for an assay is provided, including: (a) an interface for receiving and operating on a cartridge; (b) logic for causing the following operations to be performed in the cartridge: receiving an input sample comprising DNA; directing a first portion of DNA from the input sample to a first PCR chamber of the cartridge; directing a second portion of DNA from the input sample to a second PCR chamber of the cartridge; initiating a qPCR on the first portion of DNA from the input sample in the first PCR chamber; initiating a STR PCR on the second portion of DNA from the input sample in the second PCR chamber; detecting an output signal from the first PCR chamber, which provides information about DNA concentration in the first portion of DNA from the input sample; and completing the STR PCR by conducting a predetermined number of amplification cycles on the second portion in the second PCR chamber, wherein the predetermined number of amplification cycles is based on the output signal from the first PCR chamber.

In another aspect of the embodiments herein, a system for preparing a sample for an assay is provided, including: (a) a plurality of interfaces for receiving and operating on a respective plurality of cartridges; (b) logic for causing the following operations to be performed in the cartridge: receiving an input sample comprising DNA; directing a first portion of DNA from the input sample to a first PCR chamber of a first cartridge; directing a second portion of DNA from the input sample to a second PCR chamber of a second cartridge; initiating a qPCR on the first portion of DNA from the input sample in the first PCR chamber; initiating a STR PCR on the second portion of DNA from the input sample in the second PCR chamber; detecting an output signal from the first PCR chamber, which provides information about DNA concentration in the first portion of DNA from the input sample; and completing the STR PCR by conducting a predetermined number of amplification cycles on the second portion in the second PCR chamber, wherein the predetermined number of amplification cycles is based on the output signal from the first PCR chamber.

For the avoidance of doubt, it is again noted that any of the foregoing, following, or other aspects, embodiments, or implementations of the present disclosure or invention(s) thereof, whether product (apparatus, system, kit, etc.) or process (method, step, etc.), can include (i) one cartridge having a plurality of fluid channels and a plurality of fluid chambers and/or (ii) a plurality of cartridges, each having one or more of the plurality of fluid channels and the plurality of fluid chambers.

These and other features of the disclosure will be presented in more detail below with reference to the associated drawings.

### BRIEF DESCRIPTION OF THE DRAWINGS

Figure 1A is a flow chart depicting a general approach to DNA sample preparation by performing qPCR and STR PCR on a single cartridge.
Figure 1B is a flow chart depicting a general approach to DNA sample preparation by performing qPCR and STR PCR on different cartridges.
Figure 2A is an illustration of a sample cartridge containing various chambers, inlets, and fluidic connections suitable for enabling parallel performance of a qPCR and an STR PCR.
Figure 2B is an illustration of a reagent vial suitable for delivering reagent solution to a cartridge such as the sample cartridge of Figure 2A.
Figures 3A and 3B are illustrations of optical components that may be used to produce and read optical signals from a qPCR reaction on a cartridge such as the sample cartridge of Figure 2A. The optical components of Figures 3A and 3B may be integrated into an instrument that interfaces with a cartridge to control flow and reactions on the cartridge.
Figure 4 is a flow chart illustrating an example process, including qPCR and STR PCR that may be performed on an instrument such as the sample cartridge of Figure 2A.

### DETAILED DESCRIPTION

### Context and Overview

Processing DNA samples taken from individuals, in person, presents relatively few challenges. For example, a buccal swab sample of cheek cells taken from an individual onsite provides an amount of DNA that is reasonably well controlled. Crime scene samples, by contrast, are not at all well controlled. Often they are taken from an unknown person and might even include DNA from multiple individuals, some of whom might be nonhuman. And, different crime scene samples can have widely varying amounts of DNA, with concentrations varying multiple orders of magnitude from sample-to-sample. Quantities of DNA in crime scene samples can span at least four orders of magnitude, e.g., from picograms to tens of nanograms. Often it is impossible to gauge, even roughly, the amount of DNA in a sample. Examples of types of crime scene DNA samples include bone shards, teeth, blood, semen, hair, cigarette butts, etc.

The amplification process (for DNA fingerprinting (e.g., STR PCR)) may require an input having a DNA concentration that falls within a limited dynamic range. If the input sample has a concentration outside this range, the PCR may not reliably amplify the sample. As a consequence, the US FBI profiler protocol for DNA forensics, "Quality Assurance Standard for DNA Databasing Laboratories," September 2011, requires DNA quantitation for all crime scene DNA samples. After sample quantitation of crime scene samples, some amount of enrichment or dilution is typically performed to adjust the sample's DNA concentration to a level appropriate to begin STR PCR. This is part of the normalization process.

In conventional approaches, after sample quantitation and concentration adjustment, a fixed volume of sample is provided for PCR. In certain cases, the fixed sample size is about 1-15 µL. In some examples, it is about 11 µL. In these conventional processes, a fixed number of PCR cycles is used; for example, 28 PCR cycles.

Aspects of this disclosure pertain to performing DNA assays (e.g., electrophoretic STR assays). Further aspects of this disclosure pertain to performing sample quantitation and sample amplification in a sample cartridge. The resulting amplified sample may be provided to a region of the cartridge or to a different device for performing a DNA assay (e.g., capillary electrophoresis). Further aspects of the disclosure pertain to performing sample quantitation during STR PCR. Also, in certain implementations, samples are not normalized prior to performing STR PCR, at least not in a manner that requires a sample volume change such as diluting or concentrating. In certain embodiments, qPCR and STR PCR are performed on the same cartridge, optionally at the same time (or overlapping in time) and optionally using some or all of the same PCR apparatus. This allows quantitation of the STR PCR sample without substantially delaying the STR PCR process. In certain embodiments, a fixed volume sample, e.g., about 1-15 µL, is used for each of the qPCR and STR PCR. A separate volume of PCR reagents are also used (e.g., about 5-20 µL for each reaction).

As used herein a cartridge is a structure configured for use in a larger system or instrument for performing one or more biological assays, procedures, or processes and having at least one fluidically accessible processing chamber or device. Frequently, a cartridge has at least two fluidically connected chambers or devices to which access is controlled by valves or other control features. In some embodiments, two or more chambers can be disposed on two or more (separate and/or distinct) cartridges. In certain embodiments, the chambers or devices are designed or configured for sample preparation, sample processing, sample assaying, and/or related use. In various embodiments, the cartridge contains a sample receiving feature and a DNA amplification feature.

In certain embodiments, a cartridge supports two sequential process operations on a sample. Examples of such sequential operations include sample reception and cell lysis or DNA extraction; DNA extraction and DNA amplification; and DNA amplification and analysis. In an example described in more detail herein, a cartridge includes two PCR chambers for conducting parallel PCR reactions.

The cartridge is typically a unitary device that may be portable. In various embodiments, a cartridge is configured for insertion into an instrument that interfaces with the cartridge. In some cases, the interfacing provides elements or interactions for fluidically moving components through the cartridge. In some cases, the interfacing provides probing, interrogating, or reading information from the cartridge. One type of interfacing involves electromagnetic interactions with the cartridge such as stimulating a fluid with an optical signal, reading fluorescent signals, etc. For example, the interfacing may produce and read qPCR signals or capillary electrophoresis signals. Another type of interfacing involves reading a code on the cartridge such as a bar code or an RFID code.

While much of the discussion herein uses cartridges as examples of components for conducting parallel qPCR and STR PCR (overlapping in time), the disclosure is not limited to such implementations. For example, parallel qPCR and STR PCR may be conducted in a single instrument but not on the same cartridge. An instrument may be configured to conduct one or more sample processing and assaying functions, but contains separate structures, including in some cases separate cartridges, for conducting qPCR and STR PCR.

As mentioned, PCR amplification efficiency is typically less than 100%, which can influence DNA concentration as calculated using qPCR. However, when qPCR and STR PCR are performed under the same conditions, and optionally at the same time, such inefficiency may occur substantially equally in the two PCRs, thereby allowing DNA normalization without explicitly correcting these inefficiencies. Other sources of error such as partial inhibition by non-targeted DNA (load DNA), proteins, as well as other inhibitors are also be shared between the two PCRs and may affect the two reactions similarly and hence yield a reasonably correlated output.

While much of the description herein refers to STR PCR as the amplification process that benefits from qPCR, other types of target sequence (non-STR sequences) may be characterized and amplified by the methods and apparatus of this disclosure. For example, Single Nucleotide Polymorphism ("SNP") PCR and other non-STR allele amplification processes may be used. Therefore references to STR PCR may be construed to include amplification of various types of nucleic acid sequences/segments, and not be strictly limited to STR PCR. In some cases, the term "target PCR" is used to describe the PCR in which the number of amplification cycles is determined using information from qPCR. Target PCR includes STR PCR, SNP PCR, and other PCRs performed to amplify specified targets present in DNA samples. Targets are often loci of polymorphisms. In various embodiments, the amplicons of a target PCR are assayed. Example assays include electrophoresis, microarray assaying, filter binding assays, and the like.

### Pre-assay Sample Preparation without Normalization, Dilution, Concentration, Division, and/or Pre-amplification Quantitation

In some cases, there is also no diluting or concentrating after PCR; in other words, the sample volume is substantially fixed during and after STR PCR. In such cases, the only or primary adjustable process parameter may be limited to the number of STR PCR cycles to be performed.

In certain embodiments, the process simply takes a sample as is, which might have received some preprocessing such as lysis and DNA separation or extraction using magnetic beads or some other separation mechanism, but no initial quantitation is performed and no initial volumetric change such as dilution or concentration is performed. As indicated, processes such as dilution and metered fluid delivery are complex and difficult to engineer in a microfluidics apparatus or cartridge. In some implementations, to promote rapid sample analysis, the process starts with as concentrated a sample as possible, thereby reducing or minimizing the total number of STR PCR cycles.

While in various embodiments, no pre-amplification quantitation is performed, the sample may be amplified in parallel with qPCR, which can determine sample concentration on the fly as the STR PCR amplification is performed. The qPCR information so obtained is used to determine how many total cycles are necessary. This information can then be used to determine how many more cycles, if any, are necessary to sufficiently amplify the sample via STR PCR.

In various embodiments, the window of acceptance (as expressed as a dynamic range) of the downstream analysis process, such as capillary electrophoresis, is larger than the difference in concentration before and after a single cycle of amplification (i.e., approximately two times). In such embodiments, any input concentration can be made to produce an adequate output concentration if subjected to an appropriately tailored number of amplification cycles. No sample volume change is needed. For example, STR PCR may be used as the only control feature-not dilution, concentration, dividing, etc.-necessary to take an arbitrary input sample and provide it in a form suitable for downstream analysis (e.g., having a DNA concentration within the window of acceptance). This approach may also address a common concern in conventional sample processing: over-amplification, which may create artifacts rendering the sample unsuitable.

In some cases, the input to STR PCR must have less than a maximum DNA concentration; otherwise the PCR polymerase will be ineffective. Various techniques may be employed to limit the maximum concentration or clean-up the sample before amplifying. For example, a DNA extraction step may be designed so that it can capture no more than a safe amount of DNA for provision to STR PCR. In such cases, a limited quantity of the DNA capture moiety (magnetic beads, etc.) is provided upstream of the PCR process, thereby limiting the total quantity of DNA provided to the PCR chamber. In concentrated input samples, this effectively reduces the total amount of DNA in the fixed volume provided to the PCR chamber. Another potential advantage of performing DNA clean-up before amplifying is to remove inhibitors that would otherwise interfere with the amplification.

### Parallel PCR - performing qPCR and STR PCR concurrently

As indicated, qPCR and STR PCR may be conducted at the same time, or at least overlap in time so that STR PCR begins prior to concluding qPCR. In other words, the process may detect DNA concentration in a sample by temporally conducting qPCR in parallel with STR PCR. Using the information derived from qPCR, a predetermined or required number of STR PCR cycles may be determined or adjusted after STR PCR has started. For a sample having a relatively low starting DNA concentration, the process determines that relatively more STR PCR cycles are preferred or required. By contrast, for a sample having relatively higher starting DNA concentration, the process determines that relatively fewer amplification cycles are preferred or required. Regardless of the sample's initial DNA concentration, the total number of STR PCR amplification can be determined on the fly, during STR PCR.

By conducting the PCR processes in parallel, the quantitation necessary to meet standard protocol requirements is met without significantly slowing the overall process. It is of course possible to perform qPCR to completion and then begin STR PCR, and some embodiments herein employ this sequence. However, when the two processes share aspects of the process such as a single thermal profile, they can benefit from being conducted concurrently.

In terms of processing time, it may be beneficial to conclude STR amplification as early as possible by, for example, not running qPCR to full completion (which is typically about forty cycles). Rather, the qPCR is just run to a point where a preliminary reading of DNA concentration can be made. Typically, this is a few cycles beyond the cycle where a qPCR signal is first observed (sometimes referred to as the cycle threshold, Cₜ). In other words, the qPCR may be terminated early; it need not go to the full forty cycles (or whatever number of cycles is recommended for the particular qPCR process).

In some embodiments, qPCR and STR PCR are started at the same time. In other embodiments, qPCR is started earlier than STR PCR. For example, the qPCR process may start three cycles, or five cycles, or ten cycles, or fifteen cycles prior to the first cycle of STR PCR. Example embodiments presented below illustrate ways to stagger the two PCR processes.

In some embodiments, where a very high end-point STR concentration is desired, the STR PCR may start before the qPCR begins.

### Performing qPCR and STR PCR in the same cartridge

In some aspects of this disclosure, qPCR and STR PCR are performed in close proximity to one another, e.g., in the same system such as on the same cartridge. In embodiments where the qPCR and STR PCR are performed in close proximity to one another, they may share certain resources such as particular reagents that are not specific to one type of PCR or the other (e.g., buffer and nucleotides). Further, they can share a thermocycler and associated heat spreader. As a consequence, the amplification processes in qPCR and STR PCR correlate well. This is not the case when running two reactions (qPCR and STR PCR) separately in two different machines at different times, even with appropriate normalizing steps.

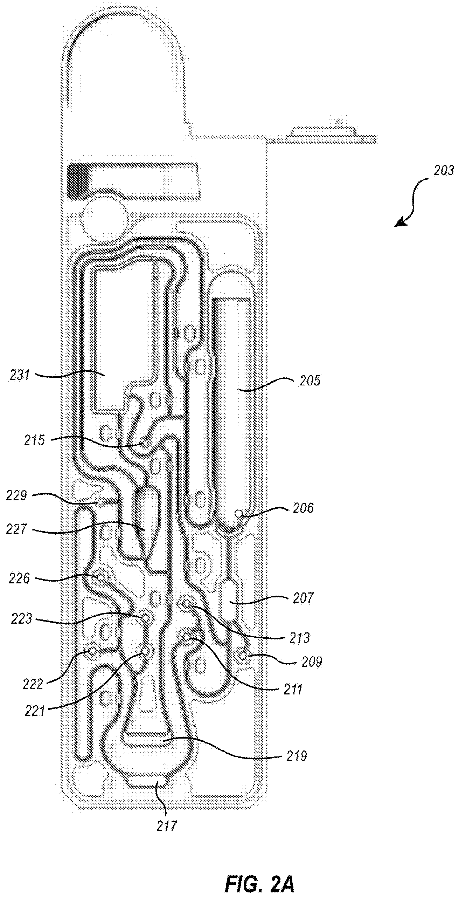

In certain embodiments, a cartridge for preparing DNA samples for assaying has two chambers: one chamber for performing qPCR and a second chamber for performing STR PCR. In certain embodiments, the two chambers of the cartridge share a single thermocycler and associated heat spreader. Because all properties of the thermal control are shared between the two chambers, the system provides enhanced correlation in amplification efficiency and hence amplicon concentration in the two chambers. In some cases, to optimize the PCR processes for qPCR and STR PCR, two thermocyclers are employed, one for the qPCR chamber and a different one for the STR PCR chamber. An example of a suitable multi-chamber cartridge design is presented below with reference to Figure 2A.

While the reactions performed in close proximity may be performed at the same time, they need not be. In some cases, the two PCR amplifications are performed serially in the same reaction chamber and optionally use some of the same reagents.

### General Process Flow

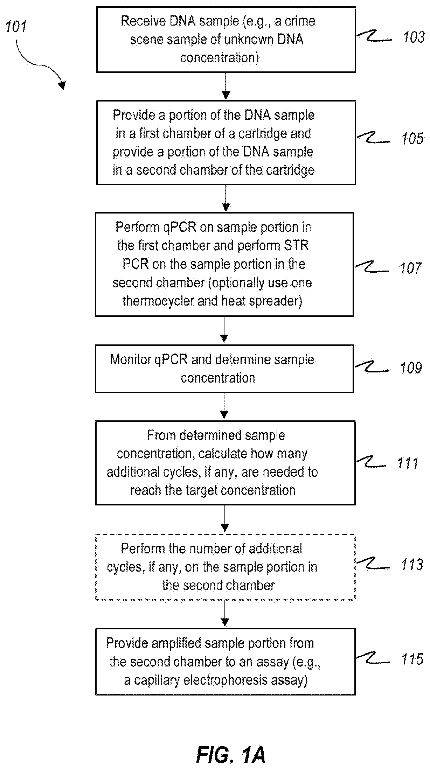

Figure 1A depicts an example process flow 101 in accordance with certain embodiments of this disclosure. The process begins with an operation 103 where a sample cartridge receives a DNA sample. In certain embodiments, the sample is a crime scene sample having an unknown DNA concentration. After receiving the sample in, for example, a compartment of a sample cartridge, the sample is optionally preprocessed in a manner not depicted in this flowchart. For example, the sample may be exposed to a lysis buffer and/or the DNA in the sample may be enriched. Regardless of these optional steps, after receiving the DNA sample, it is divided to provide the first portion to a first chamber and the second portion to a second chamber. In certain embodiments, these chambers are distinct PCR chambers of the sample cartridge. In the flowchart, see operation 105.

After dividing the input DNA sample in a manner described, the system performs qPCR on the sample portion in the first chamber and it performs STR PCR on the sample portion in the second chamber. See operation 107 of the flowchart. Optionally, the system uses a single thermocycler and heat spreader to perform both qPCR and STR PCR in the first and second chambers, respectively.

As the qPCR is performed, the system monitors its progress and ultimately determines the DNA sample concentration or otherwise obtains information relating to quantitation of the DNA sample. See operation 109 in flowchart 101. Using information about the DNA concentration, the system calculates how many cycles are needed to reach the target concentration in the other chamber, via STR PCR. See operation 111. If necessary, system performs the number of additional cycles identified in operation 111. These additional cycles are performed on at least the portion of the sample in the STR PCR chamber. See optional operation 113 in flowchart 101.

Finally, after conducting STR PCR according to the number of cycles determined in operation 111, the system provides the amplified STR sample portion from the second chamber to an assay. As explained, one example of an assay is a capillary electrophoresis assay. See operation 115 of flowchart 101.

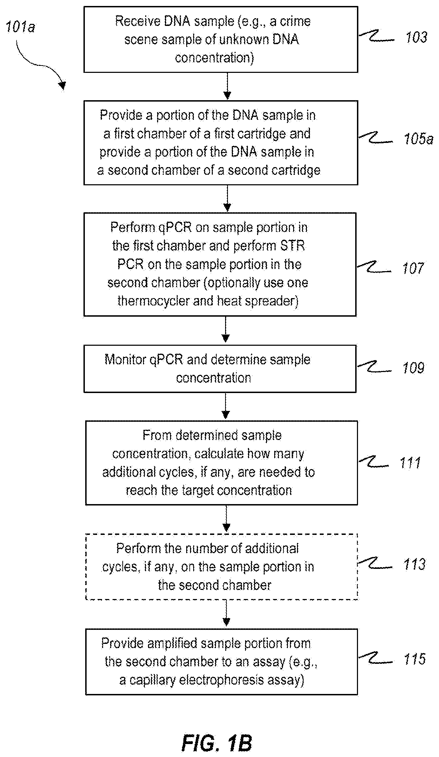

Figure 1B depicts an example process flow 101a in accordance with certain embodiments of this disclosure. The process is substantially similar to process 101 depicted in Figure 1A. However, in process 101a, a first portion of the DNA sample is provided in a first chamber of a first cartridge and a second portion of the DNA sample is provided in a second chamber of a second cartridge. In the flowchart, see operation 105a. Accordingly, in some embodiments, the first and second chambers are distinct PCR chambers of separate sample cartridges, such that qPCR and STR PCR are performed in separate chambers of respective separate cartridges.

### Example Embodiments

A few example embodiments will now be presented. The disclosure is not limited to these embodiments.

Embodiment 1: In some implementations, the two reactions (qPCR and, e.g., STR PCR) are started simultaneously and the STR PCR is performed for at least the number of cycles used for qPCR. If qPCR indicates that the current DNA concentration (after a number of cycles sufficient to quantitate) is sufficient for assaying, the STR PCR ends. If on the other hand qPCR indicates that the current DNA concentration is insufficient, a determined number of additional STR PCR cycles are performed.

In some implementations, the qPCR and STR PCR share resources; e.g., they are performed on a single cartridge. In such embodiments, the end concentration in the STR PCR chamber will correlate well with that of the qPCR, regardless of the initial sample DNA concentration. This produces a sample having an accurately known final DNA concentration suitable for down-stream detection. In some implementations, the qPCR and STR PCR are performed in the same instrument but not necessarily on the same cartridge. For example, the qPCR and STR PCR may run in two microtubes, optionally side-by-side, in an instrument. The total number of STR PCR cycles, or an additional number of STR PCR cycles after qPCR completes are determined based on the readout from the qPCR.

In some implementations, the two chambers are simply subjected to the number of cycles needed to detect the product in the qPCR chamber and then a few additional cycles to reach the desired end concentration (based on the concentration determined when qPCR detection begins). The number of additional cycles depends on the desired end concentration, which may be experimentally pre-determined. In some cases, the number of additional cycles is between about two and ten additional cycles.

Embodiment 2: Unlike embodiment 1, the STR PCR and the qPCR processes may be started at different times, although they overlap. In some implementations, the qPCR starts earlier, in effect giving the qPCR a head start. One reason for this is that qPCR often requires many cycles to reach a concentration that can be detected and used for quantitation, and hence this may take more than the cycles that is needed for the STR amplification to reach its target concentration. For example, as many as forty cycles may be performed. In some cases, if the STR sample is subjected to all the cycles necessary to provide a reliable qPCR reading, the STR sample's final concentration may be too high for assaying and/or have been over-amplified to the point of developing artifacts. Even if qPCR is not run to its full recommended completion (e.g., forty cycles), its limit of detection might require more cycles than necessary to reach the desired end concentration in the STR chamber.

Thus, it can be desirable for the qPCR process to start somewhat ahead of the STR PCR process. For example, the qPCR may begin one to about fifteen cycles (or about five to about ten cycles) before the STR PCR.

Delaying STR PCR may be accomplished in various ways. In this embodiment, the system delays introduction of the STR PCR sample to its cartridge chamber. This approach may require a separate holding chamber (e.g., in the cartridge) for the STR portion of the sample.

The length of delay is typically measured in numbers of PCR cycles and may be chosen based on a conservative estimate of the sample's initial DNA concentration. For example, if the estimate suggests that only thirty PCR cycles will be necessary to reach the assay's target concentration, and assuming that qPCR requires thirty cycles, the process may delay about ten cycles before introducing sample to the STR PCR to the STR chamber. If, at thirty cycles, qPCR shows that STR PCR requires a total of twenty-three cycles, then STR PCR can continue another three cycles to reach the desired concentration. Note that the higher the input concentration, the earlier the qPCR result. Thus, assuming a correctly chosen head start (number of cycles that qPCR starts before STR PCR), the qPCR result will always be there on time. If a protocol employs a constant head start, then no estimation is needed.

In certain variant embodiments in which the qPCR cycles are significantly faster than the STR PCR cycles, the STR PCR may be started first or at the same time as the qPCR. At some point during the course of STR PCR, the qPCR cycle count will surpass the STR PCR cycle count. Thereafter, the qPCR will be able to report the sample concentration in time for the STR PCR process to be terminated at a point where the STR DNA has reached a desired concentration. As with Embodiment 1, Embodiment 2 may perform qPCR and STR PCR in a single instrument, and optionally on a single cartridge.

Embodiment 3: In certain implementations, the temperature protocol or other process conditions differs between the qPCR and STR PCR amplification processes. For example, the temperature of denaturing or annealing steps may be different for the qPCR and STR PCR amplifications. In such cases, the system may employ two separate sets of temperature controllers, one for each chamber, rather than one shared. The hardware may be programmed or otherwise configured to control the amplification processes differently for the qPCR and STR PCR. For example, the durations and temperatures of the annealing, chain extension, and/or denaturing operations may be different for qPCR and STR PCR. This approach allows the cycling protocols to be separately optimized for qPCR and STR PCR. This embodiment may be implemented when the qPCR and STR PCR process are implemented concurrently. As with Embodiment 1, Embodiment 3 may perform qPCR and STR PCR in a single instrument, and optionally on a single cartridge.

Embodiment 4: As with Embodiment 2, the limit of detection of the qPCR subsystem is such that the desired end concentration of DNA in the STR chamber is likely to be reached before the product in the qPCR chamber has reached a concentration suitable for down-stream detection. In this embodiment, however, introduction of sample to the STR chamber is not delayed but rather amplification in the STR chamber is inhibited for the first few cycles. In some implementations, the inhibition is accomplished by delaying adding amplification reagents to the sample. For example, if it is believed that, for any sample having an (unknown) input concentration (within an expected range), the qPCR will need no more than ten additional cycles (to reach the concentration which can be detected) than the STR PCR to reach the target concentration, the process may inhibit PCR in the STR chamber by about ten cycles. As with Embodiment 2, if at forty cycles, qPCR shows that STR PCR requires a total of thirty-three cycles, then STR PCR can continue another three cycles to reach the desired concentration. Embodiments that delay STR PCR by controlling reagent delivery, rather than controlling when the STR sample is introduced to a PCR chamber, may not require a separate STR sample holding chamber (and associated valves) upstream from the PCR chamber. As with Embodiment 1, Embodiment 4 may perform qPCR and STR PCR in a single instrument, and optionally on a single cartridge.

Embodiment 5: As with Embodiment 2, the limit of detection of the qPCR subsystem is such that the desired end concentration in the STR chamber is reached before the product in the qPCR chamber has reached a concentration suitable for down-stream detection. The system may be designed such that a larger volume of sample is used for the qPCR chamber than the STR PCR chamber (assuming equal volume of the chamber, which should benefit their correlation). As with Embodiment 1, Embodiment 4 may perform qPCR and STR PCR in a single instrument, and optionally on a single cartridge.

Embodiment 6: In some implementations, the qPCR and STR PCR processes are performed fully serially, such that the qPCR reaction is first run to a point necessary to determine the number of cycles needed for a later STR PCR reaction, which is only initiated after the qPCR results are interpreted to show how many STR PCR cycles are needed. The qPCR and STR PCR processes may be performed in different chambers of a cartridge. The qPCR and STR PCR processes may be performed using process conditions optimized for their respective amplifications. For example, each may employ its own sequence of temperatures in a cycle. In some implementations, the same temperature controller and/or other amplification hardware is shared between the two processes conducted in series, but operated differently for the qPCR and STR PCR. As with the former embodiment, the hardware may be programmed or otherwise configured to control the amplification processes differently for the qPCR and STR PCR. For example, the durations and temperatures of the annealing, chain extension, and/or denaturing operations may be different for qPCR and STR PCR.

Embodiment 7: This embodiment is similar to prior one, but rather than running the qPCR and STR PCR amplification processes in separate chambers, both processes are run in the same chamber. Such implementations perform the qPCR and STR PCR serially but in the same chamber, which may be part of a cartridge.

Embodiment 8: In some implementations, qPCR and STR PCR are performed in the same chamber at the same time. This is effectively a multiplex PCR process. Primers for both qPCR and the STR PCR are provided in a single sample mixture with, e.g., a crime scene sample. In certain embodiments, a single polymerase is used in the sample mixture. Probes for qPCR are included in the mixture. In some implementations, to avoid interference from fluorescent signals from qPCR probes during assaying, non-fluorescent methods of detection are used in the assays or the qPCR probes are separated from the assay sample prior to assaying. Examples of such methods include some next generation sequencing techniques and microarray techniques. However, in some embodiments, the processes employ separate dyes for qPCR and STR PCR. The qPCR nucleic acid dye is used to quantitate the sample, and the STR PCR nucleic acid dye is used to interrogate STRs during electrophoresis or other assay procedure. When two or more DNA dyes are used in the amplification mixture and the qPCR reactants are not removed or otherwise rendered non-interfering prior to the assay, they may be chosen to have emission frequencies (or other detectable frequencies) that are sufficiently distinct to allow accurate qPCR analysis. For example, the dyes may have sufficiently separate excitation or emission frequencies that the qPCR signal is not obscured by color signal from dyes used for the STR interrogation. Typically, the dyes used for STR interrogation do not employ quenchers, and hence would always emit throughout the qPCR analysis. Further, during interrogation of the STR alleles, the qPCR dye should have an emission frequency that is distinct from the any of the STR PCR dyes so that the interrogation detects only the dyes for the STR alleles.

### qPCR

Quantitative PCR, which is sometimes referred to as Real Time PCR, employs multiple loci and associated primer sequences. It also employs probes that are non-priming DNA segments having a sequence complementary to that of a PCR amplicon. Each probe includes a fluorescent dye (referred to as a donor) and an associated quencher. On the probes, the donors and quenchers are sufficiently close to one another that the donor's emission is quenched (by Fluorescence Resonance Energy Transfer or "FRET") and no fluorescence signal is detected in the sample. However, as amplification progresses, the polymerases will encounter probes bound to amplicon sequences, and the polymerases exonuclease activity destroys the probes and releases the donors and quenchers. As a result, the quencher becomes separated from the donor, so the donor can emit unquenched fluorescence signal. This signal becomes detectable after a number of PCR cycles that depends on the initial, unknown concentration. and becomes rapidly more intense during the exponential phase of PCR. Using appropriate modeling and calibration, the initial concentration of sample DNA can be determined by the strength and shape of the fluorescence signal. Other forms of qPCR such as those that do not employ donor-quencher pairs are known in the art and may be used in the embodiments disclosed herein.

In certain embodiments, qPCR primers are designed to amplify regions of the genome that are uniquely human. In certain embodiments, the qPCR reagents are provided in a product labelled the Quantifiler^{®} Trio PCR Amplification Kit available from Thermo Fisher Scientific of Waltham, MA.

### STR PCR

STR PCR is described in various places including J. Butler, Fundamentals of Forensic DNA Typing, Elsevier, 2009. In certain embodiments, the STR PCR reagents are provided in a product brand known as the Globalfiler^{®} PCR Amplification Kit available from Thermo Fisher Scientific. As indicated, the disclosure is not limited to STR PCR, but includes any of the various PCRs designed to amplify targets, including various loci of polymorphisms.

### DNA capture mechanisms

In certain embodiments, the cartridge or another part of the sample preparation or assaying elements includes a device or mechanism for ensuring that the DNA concentration in the PCR chambers does not exceed a threshold value. In some cases, the input to STR PCR must have less than a threshold DNA concentration; otherwise the PCR polymerase will be ineffective. Various techniques may be employed to limit the maximum concentration. For example, the DNA extraction step may be designed so that it can capture no more than a safe amount of DNA for provision to STR PCR. In other words, if a limited quantity of DNA capture moieties are provided upstream of the PCR process, the limited quantity will effectively reduce the total amount of DNA in the fixed volume provided to the PCR chamber.

### Assaying

Some embodiments employ STR electrophoresis to analyze crime scene samples (or other DNA samples for forensics and other uses). The amplified STRs are provided to a capillary where electrophoresis occurs. In certain embodiments, multiple sample preparation cartridges feed a single capillary. The capillary may be run in a staggered format to facilitate throughput. See for example US Patent Application No., 62/509,618, filed May 22, 2017. Electropherograms produced may be analyzed using various techniques such as those described in US Patent Application No., 62/432,512, filed December 9, 2016.

### Apparatus Context

In certain embodiments, STR PCR and qPCR are performed on a single sample cartridge, which may be a unitary device having recesses, chambers, or other features for holding samples and other elements. One example of a suitable sample cartridge is described below. Various recesses are provided for different stages of processing, and may be used for a number of purposes. In some implementations, the recesses are formed between a body of the sample cartridge and a deformable layer that backs the sample cartridge. During operation, some of the chambers are filled with sample and/or reagents at various stages of processing. The chambers, when opened, are in fluidic communication with fluidic channels in the sample cartridge. The chambers can be fluidically connected and then isolated from one another by actuation of valves in the sample cartridge.

The sample cartridge recesses are used for various purposes. For example, these recesses may be used to hold reagents such as nucleic acid size standards, PCR master mix, and PCR primers. In some implementations, one recess serves as a sample compartment (which may double as a lysis compartment) for an input sample such as a crime scene sample. In one example, while the swab or other substrate for carrying the sample is provided in the sample compartment, a lysing reagent may be flushed through the compartment. In certain embodiments, the sample compartment is fluidically connected to a DNA enrichment or clean-up compartment where the sample can contact magnetic beads (or other capture structures) that pull DNA from the lysed sample. In certain embodiments, the DNA enrichment compartment is fluidically connected to a wash or clean-up chamber where the magnetic beads may be washed to purify the sample or, more particularly, the DNA in the sample. This bead-based DNA capture technology can help adjust the sample concentration to a level that is reasonably close to that anticipated for the subsequent PCR reaction. After the DNA samples are extracted with the beads, they are passed through to chambers where PCR occurs. As noted, there may be two chambers that serve as PCR chambers, one for qPCR and the other for STR PCR. As explained below with reference to Figure 2A, the sample cartridge may also include another recess that serves as a waste chamber. Still other chambers may be provided for mixing, filtering, reacting, etc.

An instrument is typically provided for interacting with the sample cartridge. Together the instrument and the cartridge may provide forensic DNA profiles by generating DNA STR profiles using NDIS-approved chemistry. To operate, the user may provide an input sample in a sample cartridge, which is then inserted into a sample port on the instrument. The sample may then be processed on the sample cartridge before being routed via tubing to an assay location.

In certain embodiments, the instrument includes various features that interact with the cartridge to prepare the sample. For example, the instrument may have a sample preparation sub-system which includes at least one sample cartridge interface configured to engage a sample cartridge, sources of reagents for performing a biochemical protocol, and a fluidics assembly configured to move reagents within the sample preparation sub-system. The fluidics assembly may include a pump such as a syringe pump. The pump may be fluidically connectable through valves to the sources of reagents such as water and lysis buffer, and to a source of air. In some implementations, the pump delivers lysis buffer and water through fluidic lines to an inlet port on the sample cartridge. Air or liquid pressure can be applied by the pump to the inlet port on the sample cartridge to cause liquid to move into, within, through, or out of the sample cartridge as needed.

In certain embodiments, each of the sample cartridges interacts with the instrument through a sample cartridge interface. For instance, each of the sample cartridges can interact with the instrument through a respective sample cartridge interface. The sample cartridge interface may include a ram actuation assembly, a base plate, and a cover plate. A user inserts the sample cartridge into a slot formed by the base plate and cover plate. The ram actuation assembly may include a cam array, a ram array, and an array of biasing elements. These elements work together such that each ram engages a valve of the sample cartridge. A top piece of the sample cartridge interface includes apertures for pistons or plungers configured to actuate ball valves to chambers connected to ports, and apertures through which fluid conduits pass to mate with input and output ports on the sample cartridge.

In certain embodiments, a base plate and a cover plate of the instrument's sample cartridge interface together form a guide that engages with and guides the sample cartridge when the cartridge is inserted into the sample cartridge interface. The sample cartridge interacts with the sample cartridge interface as described above. For instance, the sample cartridge interface actuates rams which engage with valves on the sample cartridge to cause changes within the sample cartridge as needed. The valves control the liquid flow along the various channels in the sample cartridge through temporary deformation of the deformable layer on the sample cartridge at particular valve locations, and/or through plungers that push open a ball valve. The sample cartridge interface includes appropriate apertures for accommodating the rams, pistons, plungers, fluidic lines, etc. which interact with the sample cartridge.

In certain embodiments, a back end cartridge interacts with the instrument through a back end cartridge interface (sometimes referred to as an electrophoresis cartridge interface). The back end cartridge interface is configured to releasably engage with the back end cartridge. When inserted, the back end cartridge becomes fluidically connected to tubing that connects to an outlet port of the sample cartridge. In this way, the reaction product is routed from the sample cartridge to the back end cartridge where electrophoresis occurs. The back end cartridge interface also provides electrical connections between the electrodes (e.g., anode and cathode) of the back end cartridge and a power source of the instrument. The back end cartridge and back end cartridge interface further engage with one another via optical communication (e.g., optical communication between an optical window of the back end cartridge and an optics module of the instrument), thermal communication (e.g., between the electrophoresis capillary and a thermal control assembly of the system), mechanical communication (e.g., between an anode sub-assembly of the back end cartridge and a mechanical interface in the instrument, and between a cathode sub-assembly of the back end system and a mechanical interface in the instrument), electrical communication, magnetic communication (e.g., between the back end cartridge and the instrument), and/or fluidic connections such as the amplified DNA output or source of bulk reagents.

As mentioned, in certain embodiments, the instrument includes tubing used to deliver a reaction product from the sample cartridge (where the sample is prepared/processed for analysis) to the back end cartridge (where the reaction product is analyzed via capillary electrophoresis). The transfer of sample through the tubing is controlled by the instrument such that the reaction product is routed through the various elements of the system, as needed.

In the embodiment depicted in Figure 2A, the PCR chambers have smile shapes. In other embodiments, the PCR chambers have other shapes such as circles, ovals, rectangles, or other polygons. Once a portion of the sample is in one of the PCR chambers, it is optionally held in place by a small piece of paper or other absorbent material that may appear as a dot in the cartridge. While adhered to this region, the sample DNA is exposed to reagents suitable for conducting PCR. These reagents include primers for the loci to be amplified, appropriate polymerase, buffer, nucleotides, etc. In some embodiments, the sample cartridge will have at least two reagent chambers or at least two reagent inlets to the PCR chambers. Different reagents are provided to the STR PCR chamber and the qPCR chamber. The STR PCR chamber has an outlet for providing amplified STR sample to other assaying components such as an input region for a capillary where electrophoresis will be performed. As mentioned, electrophoresis may be performed on a separate cartridge, although this is not a requirement. The qPCR chamber need not have an outlet, although it may be connected to, for example, a waste chamber.

The PCR chambers in the cartridge may thermally contact a region of the instrument where one or more thermocyclers can apply desired PCR heating profiles. In some embodiments, the cartridge fits between two elements that together execute and apply thermocycler temperature profiles: one is a heat spreader and the other is a thermal element that drives the temperature variations for thermal cycling. In certain embodiments, the heat spreader is provided in the cartridge.

In certain embodiments, a single thermocycler is used for both the qPCR and the STR PCR chambers. When a single thermocycler apparatus is employed, which includes a single heat spreader and controller, the temperature profile must be able to perform both qPCR and STR PCR. In certain embodiments, the thermocycler thermal profile is optimized for the STR PCR reaction, or at least biased towards it. This is for two reasons. First, while certain standards for DNA fingerprinting require some quantitation, they do not necessarily specify any level of accuracy. Given that there is no specified level of accuracy-e.g., to within 2% of the actual concentration-the level of accuracy for qPCR process can be relatively rough. See the protocol described in "Quality Assurance Standard for DNA Databasing Laboratories," September 2011. Second, the STR PCR must be conducted with a temperature profile that allows relatively long alleles to be amplified. Thus, the temperature and duration of the denaturing, chain extension, and annealing operations are set for amplifying STR alleles, which may be greater than needed for the qPCR alleles.

In certain embodiments, the instrument includes one thermocycler for the qPCR chamber and a separate thermocycler for the STR PCR chamber. These thermocyclers may be independently controlled as appropriate when the temperature profile applied for qPCR is somewhat different from that optimized for STR PCR. This may be appropriate to optimize the PCR thermal profiles for qPCR and STR PCR respectively. The different allele loci and allele size between the qPCR amplicons and the STR amplicons give rise to different optimal thermal profiles. The allele lengths can vary considerably for the STR PCR such that the longest alleles are most likely to be much longer than the longest loci to be amplified in qPCR. Therefore, the denaturing and chain extension steps may need to have a longer duration in STR PCR amplification than in qPCR amplification.

### Example DNA sample processing cartridge

Figure 2A depicts a cartridge 203 that may be used in various embodiments described herein. As shown, cartridge 203 contains various compartments or chambers and fluidic lines associated with sample reception and preparation. Cartridge 203 contains a sample chamber 205 shaped, sized, and/or otherwise configured to receive a sample from a pipette, a swab, or other sample delivery element. In certain embodiments, sample chamber 205 is merely an entry point in cartridge 203 for receiving a sample that is to receive subsequent processing. In other embodiments, however, sample compartment 205 is a region where one or more initial sample processing steps are performed. For example, preliminary sample processing operations, such as cell lysis and/or DNA extraction, may be performed in sample chamber 205. In one embodiment, a lysis solution is introduced into cartridge 203 from an inlet port 215, and is then transported to sample chamber 205. As shown, inlet port 215 is fluidly connected to sample chamber 205 via one or many fluid delivery lines in cartridge 203. After contacting lysis solution, cells contained in the sample are lysed and their DNA becomes available for extraction. In certain embodiments, a DNA capture reagent such as nucleic acid probes coupled to magnetic beads is introduced into sample chamber 205 where it interacts with and ultimately captures free DNA from the sample.

In certain embodiments, only lysis or DNA extraction, but not both, are performed in sample chamber 205. In certain embodiments, neither cell lysis nor DNA extraction are performed in sample chamber 205. In some cases, one or both of these operations is carried out elsewhere on a cartridge, and, in some cases, one or both of these operations is not conducted on a cartridge.

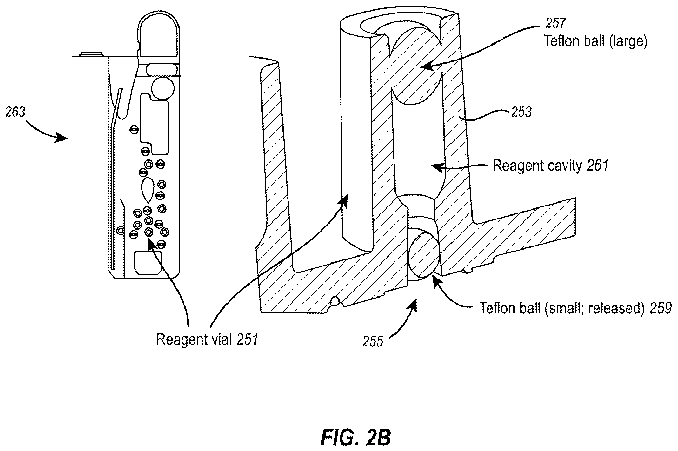

After initial processing, if any, the sample is drawn from sample chamber 205, optionally through an input port 206 into a DNA sample preparation chamber, for example, into a clean-up or wash chamber 207 that holds and dries the sample DNA before mixing it with fresh solution and ultimately releasing it. In one example, valves are set to have the vacuum applied in chamber 231, and liquid is pulled from 205 via 207 to 231. In certain embodiments, DNA clean-up chamber 207 is located next to a magnet in the instrument that engages cartridge 203. The magnet allows the extracted and captured DNA from the sample to remain fixed in place while the sample is washed or otherwise processed before amplification and/or other downstream processing. In certain embodiments, while present in DNA clean-up chamber 207, the captured DNA is allowed to dry and then become re-solubilized by introduction of water from, for example, an inlet 209. In various embodiments, an inlet to the cartridge may take the form of or include a vial or syringe configured to deliver material to the cartridge via an opening or port in the cartridge. An example, vial is presented in Figure 2B.

In embodiments employing magnetic beads or other DNA capture structure, the DNA is subsequently released to permit amplification. In some cases, the magnetic field that holds the magnetic beads holding captured DNA in place during processing in chamber 207 is released, thereby allowing the DNA to be pushed or drawn further along cartridge 203 toward amplification. In some cases, the magnet is not withdrawn; instead the DNA is released by contact with a solution providing conditions that facilitate release. In such embodiments, the eluent may come from inlet or vial 209. In certain embodiments, DNA capture and release technology includes a sequence of conditions, the first involving conditions in which the beads become positively charged and selectively bind to DNA, and the second involving conditions in which DNA is released from the beads. The conditions that may be varied include pH and/or salt concentration. For example, a solution providing the second conditions (DNA released from beads) may have a relatively low salt concentration or a relatively high pH (depending on capture mechanism). As an example, the DNA capture and release reagents may be the "ChargeSwitch"^{™} from Thermo Fisher Scientific of Waltham, MA. Other methods sometimes employ a chaotropic agent and/or a crowding agent. A common chaotropic technique uses silica beads and Guanidinium chloride. In some cases, the process employs polyethylene glycol as a crowding agent.

From DNA clean-up chamber 207, the sample DNA is provided to two separate chambers 217 and 219. For example, chambers 217 and 219 may be two separate amplification assay chambers 217 and 219 (e.g., PCR chambers 217 and 219), wherein one portion of the processed DNA sample from chamber 207 is delivered to a first chamber 217 and a remaining portion of the sample is delivered to a second chamber 219. In certain embodiments, qPCR is conducted in chamber 217 and STR-PCR is conducted in chamber 219 or vice versa; however, other types of amplification assays may be conducted in either or both chambers 217, 219. In the illustrated embodiment, DNA sample flows or moves from chamber 207 to chamber 217 and then serially flows or moves from chamber 217 to chamber 219. In other embodiments (not shown), DNA sample flows or moves from chamber 207 to chamber 217 and then flows or moves in parallel into chamber 217 and chamber 219. The serial flow configuration advantageously helps to ensure that both chambers are filled with the same sample DNA.

In certain embodiments, one or both of the PCR chambers contains a small amount of paper to capture, sample, immobilize, and/or meter out a small quantity of sample/DNA containing liquid prior to adding the reagents (e.g., premix/mastermix) and starting PCR processing. Of course, there are other mechanisms for accomplishing this such as using surface tension-based techniques (e.g., a geometric pocket or hydrophilic patch).

In addition to receiving the sample DNA, the PCR chambers 217 and 219 receive PCR reagents including PCR primers and other PCR reactants including polymerase and nucleotides (sometimes provided as a single mixture referred to as master mix). In one depicted embodiment, the PCR primers for the qPCR reaction are provided via an inlet port 211 and an inlet port 213, while the remaining PCR reagents (e.g., master mix) are provided via the other of inlet ports 211 and 213. From inlet ports 211 and 213, the PCR reagents necessary for performing qPCR are delivered to qPCR chamber 217 where they mix with the sample DNA. Both PCR reactions may employ fluorescently labeled DNA segments (the qPCR ones are initially quenched). These segments may be stored in one of the vials in each pair of vials (or inlets) 211/213 and 221/221.

As with the qPCR reaction, the STR-PCR reaction receives appropriate primers and other PCR reagents for performing amplification of the necessary STRs. In certain depicted embodiments, the PCR reagents to conduct STR-PCR may be provided via inlet ports 221 and 223, where one or more aspects of the reagents from inlet ports 221 and/or 223 may be chemically different from those provided by inlet ports 211, 213 to chamber 217. From these ports, the components are drawn into or pushed into STR-PCR chamber 219, where they mix with the sample DNA.

With the respective portions of the DNA sample provided therein, along with the other PCR reagents, PCR reactions can be conducted in both qPCR chamber 217 and STR-PCR chamber 219. In certain embodiments, the two PCR reactions overlap in time, i.e., they are performed concurrently, at least in part. In some cases, the two PCR reactions start at the same time. In some cases, the PCR reactions in each of chambers 217, 219 start at different times and/or the number of thermal cycles is different for each of chambers 217, 219. Additionally or alternatively, the PCR reactions in each of chambers 217, 219 differ from one another in other ways, for example, may differ from one another by incorporating different PCR temperature profiles, reagents, master mixes, primers, polymerases, or the like.

In certain embodiments, a first thermocycler is provided for qPCR chamber 217 and a separate, second, thermocycler is provided for STR-PCR chamber 219. With these two thermocyclers, the PCR reactions can be controlled independently, using different temperature profiles and/or different cycle times, and/or different initiation times for the first PCR cycle. In an alternative embodiment, a single thermocycler is employed for controlling the PCR reactions in each of qPCR chamber 217 and STR-PCR chamber 219. In either approach, the thermocycler or thermocyclers may be provided as part of the instrument that engages cartridge 203 during sample processing.

In certain embodiments, the delivery of primers, polymerase, nucleotides, and other PCR reagents for amplifying the sample DNA is delayed to STR PCR chamber 219. In other words, before the PCR reagents are delivered to STR-PCR chamber 219, corresponding PCR components are delivered to qPCR chamber 217. Thus, in certain embodiments, PCR reagents are fully provided and available in qPCR chamber 217 before they are available in STR-PCR chamber 219. In this manner, the qPCR reaction can begin before the STR-PCR reaction begins. This may be the case even if a single thermocycler is used for both the quantitative and STR-PCR reactions.

A staged-delivery of PCR reagents to chambers 217 and 219 may be controlled by operation of inlet ports 211, 213, 221, and 223. In other words, inlet ports 211 and 213 can be opened and available for delivering PCR reagents to qPCR chamber 217 before inlet ports 221 and 223 are opened and available for delivering PCR reagents to STR PCR chamber 219.

In certain embodiments, using staged delivery of PCR reagents or some other staging technique, the qPCR reaction begins a defined number of cycles before the STR-PCR reaction begins. For example, the qPCR reaction may begin about five to ten cycles prior to the STR-PCR reaction. Note that when using staged-delivery of reagents, the sample may be initially provided to both PCR chambers, but reagents are flowed into the STR-PCR chamber 219 only after a few initial cycles are conducted in the qPCR chamber 217.

Note that if the STR-PCR reaction is to be delayed, it can be delayed by mechanisms other than delaying delivery of reagent. For example, the STR-PCR reaction may be delayed by simply delaying delivery of the sample DNA to the STR-PCR chamber 219. In another example, the STR-PCR reaction is delayed by controlling qPCR and STR-PCR thermocyclers separately such that the qPCR thermocycler initiates qPCR one or more cycles before the STR-PCR thermocycler initiates STR-PCR.

In certain embodiments, qPCR chamber 217 is used to determine one or more parameters for a process in STR-PCR chamber 219. For example, using the output from the qPCR reaction taking place in chamber 217, the sample processing logic of the instrument can determine, explicitly or implicitly, the concentration of the sample DNA. Using this concentration information, the sample processing logic may determine a number of cycles of STR-PCR amplification to provide an STR sample at a concentration within a range specified for further analysis, such as capillary electrophoresis. Note that the instrument logic need not actually calculate or otherwise determine the DNA concentration-whether of the sample as received or at some point during amplification or other processing-when determining a number of cycles of STR PCR.

After STR loci of the DNA sample are amplified, the STR-amplified sample is pushed or drawn into a mix chamber 227 where it may be mixed with one or more components for further analysis such as DNA fingerprinting. For example, in mix chamber 227, the STR-amplified DNA sample may be mixed with an internal lane standard (ILS) suitable for conducting DNA fingerprinting via capillary electrophoresis. From mix chamber 227, the sample is provided to an outlet port 229 where it exits sample preparation cartridge 203 and is made available to a downstream processing device such as a capillary electrophoresis cartridge.

Sample preparation cartridge 203 additionally includes a waste chamber 231 which collects waste from one or more of the processing operations described herein.

Figure 2B shows an example of a reagent vial that may be used to deliver reagents to a cartridge via appropriate inlets or ports. For example, a reagent vial may be employed to deliver water, such water having a low salt concentration, via inlet 209, or PCR reagents such a primers and master mix via inlet ports 211, 213, 221, and 223. As shown in Figure 2B, a reagent vial 251 includes a generally tubular wall 253 that funnels into an outlet 255 that may couple to a cartridge via a port or inlet in the cartridge. The tubular wall encloses a cavity 261 where reagent resides until dispensed. Within the reagent vial are a larger ball 257 and a smaller ball 259, proximate the outlet 255. The smaller ball 259 is released when the larger ball 257 is pushed. This action, which may be initiated by engagement with a push rod or other structure in the instrument interfacing with the cartridge, dispenses reagent out of the vial and into the cartridge. Figure 2B also depicts a cartridge 263 having various recesses or chamber, inlets, and outlets. As illustrated, reagent vial 251 engages an inlet on the surface of cartridge 263.

In some examples, the sample chamber is configured to capture about 5-5000 uL of sample. As a further example, the PCR chambers are each able to hold about 3-20 uL of solution for the amplification reactions. As a further example, reagent vials, such as those that may be provided at inlets 209 and 211, may each hold about 3-15 uL of solution. As a further example, a mix chamber is sized to hold about 20 - 1000 uL of solution. As a still further example, a diluent chamber or vial 222 (e.g., for introducing an ILS) may hold about 10 - 200 uL of solution. In some embodiments, a vial 226 holds air and is used to displace all the dispensed liquid (from 221, 223, and diluent from 222) up into the mix chamber 227. Hence, in certain embodiments, the mix chamber 227 is sized to hold at least the volume of the STR PCR reactions (sample and reagent), in addition to the diluent and some volume for air bubbles.

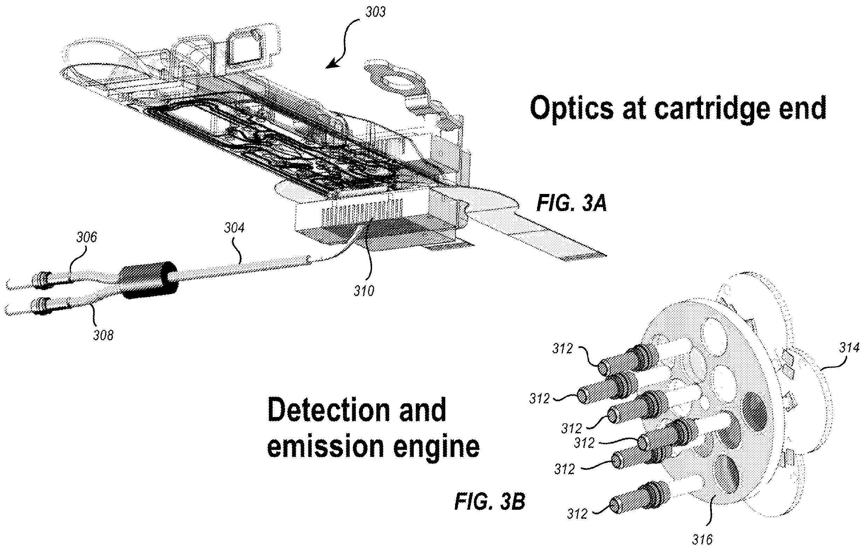

Figure 3A illustrates a sample preparation cartridge 303 when installed in an instrument configured to receive and optionally interpret optical signals from qPCR. Note that cartridge 303 may be identical to cartridge 203 of Figure 2A. As shown in Figure 3A, an optical transmission device 304 may include a source fiber 306 and a detection fiber 308. Note that in some embodiments, there is a lens or other optical element in the device that may be in contact with the cartridges. In certain embodiments, the elements are optically coupled to a qPCR chamber (e.g., chamber 217 of cartridge 203) via an optical coupling device 310. Other optical configurations know in the art may be utilized.

Figure 3B shows a plurality of fiber ends 312 for source and detection fibers from three different sample processing cartridges (a total of six fibers). The fiber ends provide for optical communications with sources (e.g., LEDs) and photodetectors on a board 314. A color filter wheel 316 may be provided between fiber plugs 312 and board 314.

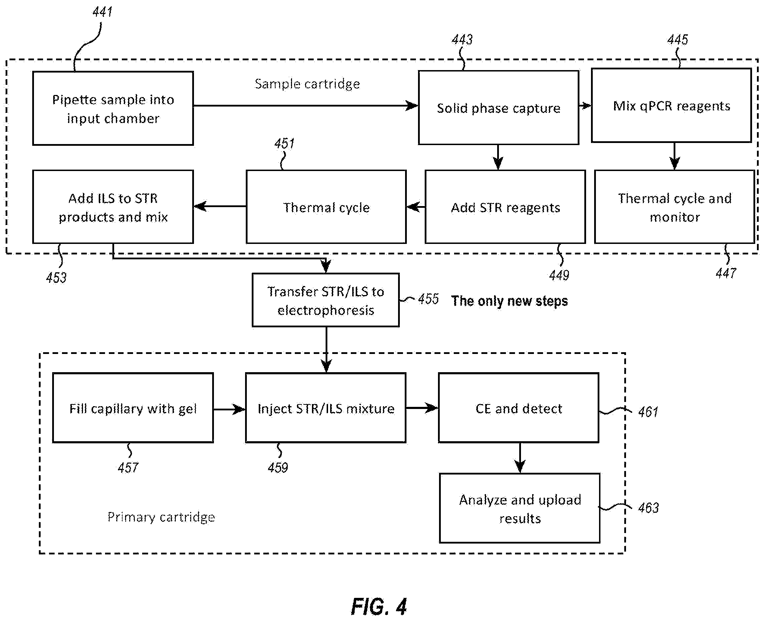

Figure 4 presents an example process flow that may be implemented on a sample processing cartridge such as cartridge 203. As illustrated, the process flow begins with pipetting a sample into an input chamber such sample chamber 205. See operation 441. Understand that other sample introduction techniques may be appropriate depending on the design of the cartridge and the type of sample.

After introduction to the cartridge and prior to PCR, the sample may be subjected to a DNA capture technique such as a magnetic bead-based technique. See operation 443. More generally, this may be any technique that captures DNA from a sample to allow cleaning of the sample prior to PCR. Such capture process may be used for, e.g., purification and/or up-concentration. In certain embodiments, the process is conducted without a pre-PCR capture process.

Regardless of how the sample DNA is initially processed, it is divided into at least two portions, a first portion that is mixed with qPCR reagents (see operation 445) and a second portion that is mixed with STR-PCR reagents (see operation 449). The first portion is then thermal cycled according to a qPCR protocol to provide some indication of the quantity of DNA in the sample (see operation 447), and the determined quantity is used to determine how much amplification is needed for the second portion of the sample DNA. Thereafter or concurrently with this determination, the second portion is subjected to thermal cycling to amplify the STRs of the sample to a concentration suitable for further assaying or other analysis such as DNA fingerprinting. See operation 451. The number of thermal cycles in operation 451 is determined using the quantitative information obtained in operation 447. In some embodiments, operation 447 is not performed to the full extent (number of cycles) typically employed in qPCR protocols to provide a quantitation of DNA in a sample. For example, qPCR process may employ fewer cycles than the number of cycles conventionally used to obtain a precise DNA concentration (based on the sample's initial concentration).

The amplified STRs produced in operation 451 have a concentration suitable for analysis, but they may first be mixed with assay-specific components such as an internal ladder standard (ILS) or other size indicator used for DNA fingerprinting by capillary electrophoresis. See operation 453. The resulting mixture can then transferred to a device suitable for assaying or other analysis. See operation 455, in which the mixture is transferred to a device for electrophoresis.

In certain embodiments, operations 441-453 are performed on a single cartridge such as a sample preparation cartridge like cartridge 203. In certain embodiments, operation 455 transfers amplified STR DNA from such sample preparation cartridge to a second cartridge where DNA analysis is conducted.

Regardless of the device on which further analysis is conducted, process operations 457, 459, 461, and 463 illustrate how the amplified STR DNA may be processed for analysis. As shown, operation 457 involves filling a capillary with gel to permit capillary electrophoresis of the amplified STR sample. In operation 459, the STR/ILS mixture transferred in operation 455 is injected into the capillary. Next, in operation 461, capillary electrophoresis is performed on the STR sample, and the raw signals are detected by, for example, optical measures. Finally, at operation 463, the capillary electrophoresis signals are analyzed and the results are provided.

### Control Systems

Systems provided herein include or use computational hardware and software. In some embodiments, a system for sample preparation, amplification, and, optionally, analysis includes a controller with a processor, which may be configured as a central processing unit, memory (e.g., random-access memory and/or read-only memory), a communications interface, a data storage unit, and a display. The communications interface may include a network interface for enabling a system to interact with an intranet, including other systems and subsystems, and/or the internet, including the World Wide Web. The data storage unit may include one or more hard disks and/or cache for data transfer and storage. The data storage unit may store one or more databases, such as a relational database. In some cases, the system further includes a data warehouse for storing information, such user information (e.g., profiles) and results. In some cases, the data warehouse resides on a computer system remote from the system. In some embodiments, the system may include a relational database and one or more servers, such as, for example, data servers. The system may include one or more communication ports (COM PORTS), and/or one or more input/output (I/O) modules, such as an I/O interface.

The processor may include a central processing unit (CPU), multiple CPUs, a multicore CPU, and the like. Any processor may be employed for executing machine-readable code for implementing sample processing, analysis, and the like. Aspects of the systems and methods provided herein may be embodied in programming, sometimes referred to as logic. The processor(s) and programming or other logic may reside locally, on the instrument, or remotely, off the instrument. When the logic is located remotely it may be directly connected to the instrument, as by a local network or bus, or indirectly as by the internet. In certain embodiments, remote logic is configured as a cloud-based computational resource. In some embodiments, some of the logic resides locally and some resides remotely.

"Storage" type media may include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the internet or various other telecommunication networks. Such communications, for example, may enable loading of the software from one computer or processor into another, for example, from a management server or host computer into the computer platform of an application server. Terms such as "storage" media and computer or machine "readable medium" refer to any medium that participates in providing instructions to a processor for execution.

A machine readable medium, such as a medium that stores computer-executable code, may take many forms, including, but not limited to, various forms of tangible storage medium. Non-volatile storage media include, for example, solid state, optical or magnetic disks, such as any of the storage devices in any computer(s) or the like, such as may be used to implement the databases, etc. Volatile storage media include dynamic memory, sometimes configured as main memory of a computer platform. Common forms of computer-readable media include various forms of semiconductor, magnetic, and/or optical media.

In some embodiments, the system includes one or more computational modules for sample processing and/or analysis, each executed on the processor. The system in some cases includes instructions to cause the system to move a sample sequentially from one chamber on a cartridge to another, such as between a clean-up chamber and a PCR chamber in a sample preparation cartridge or between a sample preparation cartridge and an electrophoresis cartridge. The system in some cases includes instructions to cause the system to generate and/or read optical or other signals from a cartridge. Such signals may include fluorescent signals generated during qPCR, STR electrophoresis, and the like.

The system may be configured for extract, transform, and load (ETL) operations, which may permit the system to load information from a raw data source (or mined data) into a data warehouse. The data warehouse may be configured for use with a business intelligence system (e.g., Microstrategy.RTM, Business Objects.RTM). It also can be configured for use with a forensic database such as the National DNA Index System (NDIS)) in the USA or NDAD in the United Kingdom, State DNA Index Systems (SDIS), or Local DNA Index Systems (LDIS) or other databases that contain profiles from known and unknown subjects, forensics samples, or other sample types such as organism identifications.

### Conclusion

Systems and methods provided herein, including the components of such systems and various routines of such methods, may be combined with or modified by other systems and methods. In some situations, the above-described systems may be combined or modified by the systems described in U.S. Patent Publication No. 2011/0005932 to Jovanovich et al. ("UNIVERSAL SAMPLE PREPARATION SYSTEM AND USE IN AN INTEGRATED ANALYSIS SYSTEM") ("Jovanovich").

## Claims

1. A method of preparing a DNA -containing sample for an assay, the method comprising:
in one or more cartridges, receiving an input sample comprising DNA;
directing a first portion of DNA from the input sample to a first PCR chamber of a first cartridge;
directing a second portion of DNA from the input sample to a second PCR chamber of the first cartridge or of a second cartridge;
initiating a quantitative PCR ("qPCR") assay on the first portion in the first PCR chamber;
before completing the qPCR assay in the first PCR chamber, initiating Short Tandem Repeat ("STR") PCR on the second portion in the second PCR chamber;
detecting an output signal from the first PCR chamber, which output signal provides information about DNA concentration in the first portion of DNA from the input sample; and
completing the STR PCR by conducting a predetermined number of amplification cycles on the second portion in the second PCR chamber, wherein the predetermined number of amplification cycles is based on the output signal from the first PCR chamber.

2. The method of claim 1, wherein the input sample comprises DNA obtained at a crime scene, optionally wherein the DNA obtained at the crime scene was obtained from a source selected from the group consisting of bone, tooth, saliva, hair, blood, semen, skin, and any combination thereof.

3. The method of any of the preceding claims, wherein the one or more cartridges comprises a single cartridge optionally comprising a unitary structure comprising a plurality of fluid channels and a plurality of fluid chambers.

4. The method of any of the preceding claims, wherein neither the first nor the second portion of DNA from the input sample is diluted by adding liquid to the first or second portions of DNA from the input sample prior to initiating the qPCR or the STR PCR.

5. The method of any of the preceding claims, wherein after receiving the input sample, neither the input sample nor the second portion of DNA from the input sample are quantitated prior to initiating the STR PCR.

6. The method of any of the preceding claims, wherein, after starting the qPCR but before completing the qPCR on the first portion of DNA, initiating the STR PCR, wherein the STR PCR is initiated by providing one or more PCR reagents to the second PCR chamber and/or directing the second portion of DNA from the input sample to the second PCR chamber, optionally wherein initiating the STR PCR begins between five and fifteen PCR cycles after starting the qPCR.

7. The method of claim 6, wherein initiating the STR PCR comprises providing the one or more PCR reagents to the second PCR chamber, and optionally wherein directing the first portion of DNA from the input sample to the first PCR chamber and directing the second portion of DNA from the input sample to the second PCR chamber occur at substantially the same time.

8. The method of any of the preceding claims, further comprising, before directing the first portion of DNA from the input sample to a first PCR chamber, dividing the DNA from the input sample into the first portion and the second portion DNA, and wherein the first portion of DNA from the input sample has a volume within about twenty percent of the volume of the second portion of DNA from the input sample.

9. The method of any of the preceding claims, further comprising extracting DNA from the input sample prior to initiating qPCR on the first portion of DNA from the input sample, optionally wherein extracting the DNA comprises contacting the input sample with DNA capture beads.

10. The method of any of the preceding claims, wherein the second PCR chamber comprises an absorbent material that holds the second portion of DNA from the input sample during STR PCR.

11. The method of any of the preceding claims, wherein the qPCR and the STR PCR are performed using a single thermocycler, and wherein during at least a portion of the qPCR performed at the same time as at least a portion of the STR PCR, the qPCR and the STR PCR are subject to the same thermal profile; or wherein the qPCR and the STR PCR are performed using separate thermocyclers.

12. A system for preparing a sample for an assay, the system comprising:
(a) an interface for receiving and operating on one or more cartridges;
(b) logic for causing the following operations to be performed in the one or more cartridges:
receiving an input sample comprising DNA;
directing a first portion of DNA from the input sample to a first PCR chamber of a first cartridge;
directing a second portion of DNA from the input sample to a second PCR chamber of the first cartridge or of a second cartridge;
initiating a quantitative PCR ("qPCR") on the first portion of DNA from the input sample in the first PCR chamber;
before completing the qPCR in the first PCR chamber, initiating Short Tandem Repeat ("STR") PCR on the second portion of DNA from the input sample in the second PCR chamber;
detecting an output signal from the first PCR chamber, which output signal provides information about DNA concentration in the first portion of DNA from the input sample; and
completing the STR PCR by conducting a predetermined number of amplification cycles on the second portion in the second PCR chamber, wherein the predetermined number of amplification cycles is based on the output signal from the first PCR chamber;
and optionally, further comprising one or more actuators for dynamically controlling movement of fluid in fluidic passages of the one or more cartridges.

13. The system of claim 12, further comprising a thermocycler configured to cause the qPCR in the first PCR chamber or further comprising a thermocycler configured to (i) cause the qPCR in the first PCR chamber and (ii) cause the STR PCR in the second PCR chamber.

14. The system of any of claims 12-13, further comprising
(a) a capillary containing a separation medium and having inlet and distal ends;
(b) an inlet in communication with the capillary inlet and configured to receive samples containing DNA fragments having a plurality of different sizes;
(c) an interrogation region configured to detect DNA fragments moving through the capillary; and
(d) a power source configured to apply voltage between inlet and distal ends of the capillary.

15. The system of any of claims 12-14, further comprising a chassis enclosing the interface and at least some of the logic.

16. The system of any of claims 12-15, wherein the one or more cartridges comprises a single cartridge optionally comprising a unitary structure comprising a plurality of fluid channels and a plurality of fluid chambers.

17. The system of any of claims 12-16, wherein the logic is configured to cause the recited operations in a manner such that any one of the methods of claims 5 to 9 is performed.

18. A system for preparing a sample for an assay, the system comprising:
(a) an interface for receiving and operating on one or more cartridges;
(b) logic for causing the following operations to be performed in the one or more cartridges:
receiving an input sample comprising DNA;
directing a first portion of DNA from the input sample to a first PCR chamber of the one or more cartridges;
directing a second portion of DNA from the input sample to a second PCR chamber of the one or more cartridges;
initiating a quantitative PCR ("qPCR") on the first portion of DNA from the input sample in the first PCR chamber;
initiating a Short Tandem Repeat ("STR") PCR on the second portion of DNA from the input sample in the second PCR chamber;
detecting an output signal from the first PCR chamber, which output signal provides information about DNA concentration in the first portion of DNA from the input sample; and
completing the STR PCR by conducting a predetermined number of amplification cycles on the second portion in the second PCR chamber, wherein the predetermined number of amplification cycles is based on the output signal from the first PCR chamber.

19. The method of any of claims 1-11 or the system of any one of claims 12-17, wherein directing the second portion of DNA comprises directing the second portion of DNA from the input sample to a first PCR chamber, then from the first PCR chamber to the second PCR chamber.

20. The method of or system for preparing a sample for an assay of any of the preceding claims, wherein the assay is capillary electrophoresis of STR alleles.

## Patentansprüche

1. Verfahren zur Vorbereitung einer DNA-haltigen Probe für einen Assay, wobei das Verfahren umfasst: in einer oder mehreren Kartuschen, Aufnehmen einer DNAumfassenden Eingangsprobe;
Leiten eines ersten Teils der DNA aus der Eingangsprobe zu einer ersten PCR-Kammer einer ersten Kartusche;
Leiten eines zweiten Teils der DNA aus der Eingangsprobe zu einer zweiten PCR-Kammer der ersten Kartusche oder einer zweiten Kartusche;
Initiieren eines quantitativen PCR-Assays ("qPCR") am ersten Teil in der ersten PCR-Kammer;
vor dem Abschluss des qPCR-Assays in der ersten PCR-Kammer, Initiieren einer Short-Tandem-Repeat("STR")-PCR am zweiten Teil in der zweiten PCR-Kammer;
Detektieren eines Ausgangssignals von der ersten PCR-Kammer, wobei das Ausgangssignal Informationen über die DNA-Konzentration im ersten Teil der DNA aus der Eingangsprobe liefert; und
Abschließen der STR-PCR durch Durchführen einer festgelegten Anzahl von Amplifikationszyklen am zweiten Teil in der zweiten PCR-Kammer, wobei die festgelegte Anzahl von Amplifikationszyklen auf dem Ausgangssignal der ersten PCR-Kammer basiert.

2. Verfahren nach Anspruch 1, wobei die Eingangsprobe DNA umfasst, die an einem Tatort gewonnen wurde, wobei optional die am Tatort gewonnene DNA aus einer Quelle stammt, die aus der Gruppe bestehend aus Knochen, Zahn, Speichel, Haar, Blut, Sperma, Haut und einer Kombination davon ausgewählt ist.

3. Verfahren nach einem der vorhergehenden Ansprüche, wobei die eine oder die mehreren Kartuschen eine einzelne Kartusche umfassen, die optional eine einheitliche Struktur mit einer Vielzahl von Fluidkanälen und einer Vielzahl von Fluidkammern umfasst.

4. Verfahren nach einem der vorangegangenen Ansprüche, wobei weder der erste noch der zweite Teil der DNA aus der Eingangsprobe durch Zugabe von Flüssigkeit zum ersten oder zweiten Teil der DNA aus der Eingangsprobe vor dem Initiieren der qPCR oder der STR-PCR verdünnt wird.

5. Verfahren nach einem der vorhergehenden Ansprüche, wobei nach dem Aufnehmen der Eingangsprobe weder die Eingangsprobe noch der zweite Teil der DNA aus der Eingangsprobe vor dem Initiieren der STR-PCR quantifiziert werden.

6. Verfahren nach einem der vorangegangenen Ansprüche, wobei nach dem Starten der qPCR, aber vor deren Abschluss am ersten Teil der DNA, die STR-PCR initiiert wird, wobei die STR-PCR durch Bereitstellen eines oder mehrerer PCR-Reagenzien in der zweiten PCR-Kammer und/oder Leiten des zweiten Teils der DNA aus der Eingangsprobe in die zweite PCR-Kammer initiiert wird, wobei optional das Initiieren der STR-PCR zwischen fünf und fünfzehn PCR-Zyklen nach dem Start der qPCR erfolgt.

7. Verfahren nach Anspruch 6, wobei das Initiieren der STR-PCR das Bereitstellen des einen oder der mehreren PCR-Reagenzien in der zweiten PCR-Kammer umfasst, und wobei optional das Leiten des ersten Teils der DNA aus der Eingangsprobe in die erste PCR-Kammer und das Leiten des zweiten Teils der DNA aus der Eingangsprobe in die zweite PCR-Kammer im Wesentlichen gleichzeitig erfolgen.

8. Verfahren nach einem der vorangegangenen Ansprüche, ferner umfassend, vor dem Leiten des ersten Teils der DNA aus der Eingangsprobe zu einer ersten PCR-Kammer, das Aufteilen der DNA aus der Eingangsprobe in den ersten Teil und den zweiten Teil der DNA, und wobei der erste Teil der DNA aus der Eingangsprobe ein Volumen aufweist, das innerhalb von etwa zwanzig Prozent des Volumens des zweiten Teils der DNA aus der Eingangsprobe liegt.

9. Verfahren nach einem der vorangegangenen Ansprüche, ferner umfassend das Extrahieren von DNA aus der Eingangsprobe vor dem Initiieren der qPCR am ersten Teil der DNA aus der Eingangsprobe, wobei optional das Extrahieren der DNA das In-Kontakt-Bringen der Eingangsprobe mit DNA-Einfangbeads umfasst.

10. Verfahren nach einem der vorangegangenen Ansprüche, wobei die zweite PCR-Kammer ein absorbierendes Material umfasst, das den zweiten Teil der DNA aus der Eingangsprobe während der STR-PCR hält.

11. Verfahren nach einem der vorangegangenen Ansprüche, wobei die qPCR und die STR-PCR unter Verwendung eines einzigen Thermocyclers durchgeführt werden, und wobei während mindestens eines Teils der qPCR, der gleichzeitig mit mindestens einem Teil der STR-PCR durchgeführt wird, die qPCR und die STR-PCR demselben thermischen Profil unterworfen werden; oder wobei die qPCR und die STR-PCR unter Verwendung separater Thermocycler durchgeführt werden.

12. System zur Vorbereitung einer Probe für einen Assay, wobei das System umfasst:
(a) eine Schnittstelle zum Aufnehmen und Bearbeiten von einer oder mehreren Kartuschen;
(b) eine Logik zur Veranlassung der Durchführung der folgenden Operationen in der einen oder mehreren Kartuschen: Aufnehmen einer DNA-haltigen Eingangsprobe;
Leiten eines ersten Teils der DNA aus der Eingangsprobe zu einer ersten PCR-Kammer einer ersten Kartusche;
Leiten eines zweiten Teils der DNA aus der Eingangsprobe zu einer zweiten PCR-Kammer der ersten Kartusche oder einer zweiten Kartusche;
Initiieren einer quantitativen PCR ("qPCR") am ersten Teil der DNA aus der Eingangsprobe in der ersten PCR-Kammer;
vor dem Abschluss der qPCR in der ersten PCR-Kammer, Initiieren einer Short-Tandem-Repeat("STR")-PCR am zweiten Teil der DNA aus der Eingangsprobe in der zweiten PCR-Kammer;
Detektieren eines Ausgangssignals von der ersten PCR-Kammer, wobei das Ausgangssignal Informationen über die DNA-Konzentration im ersten Teil der DNA aus der Eingangsprobe liefert; und
Abschließen der STR-PCR durch Durchführen einer festgelegten Anzahl von Amplifikationszyklen am zweiten Teil in der zweiten PCR-Kammer, wobei die festgelegte Anzahl von Amplifikationszyklen auf dem Ausgangssignal der ersten PCR-Kammer basiert;
und optional ferner umfassend einen oder mehrere Aktuatoren zur dynamischen Steuerung der Fluidbewegung in den Fluidkanälen der einen oder mehreren Kartuschen.

13. System nach Anspruch 12, ferner umfassend einen Thermocycler, der konfiguriert ist, um die qPCR in der ersten PCR-Kammer zu bewirken, oder ferner umfassend einen Thermocycler, der konfiguriert ist, um (i) die qPCR in der ersten PCR-Kammer zu bewirken und (ii) die STR-PCR in der zweiten PCR-Kammer zu bewirken.

14. System nach einem der Ansprüche 12-13, ferner umfassend
(a) eine Kapillare, die ein Trennmedium enthält und Einlass- und distale Enden aufweist;
(b) einen Einlass, der mit dem Kapillareinlass in Verbindung steht und konfiguriert ist, um Proben aufzunehmen, die DNA-Fragmente mit einer Vielzahl unterschiedlicher Größen enthalten;
(c) eine Abfrageregion, die konfiguriert ist, um DNA-Fragmente zu detektieren, die sich durch die Kapillare bewegen; und
(d) eine Stromquelle, die konfiguriert ist, um eine Spannung zwischen Einlass- und distalen Enden der Kapillare anzulegen.

15. System nach einem der Ansprüche 12-14, ferner umfassend ein Gehäuse, das die Schnittstelle und mindestens einen Teil der Logik umschließt.

16. System nach einem der Ansprüche 12-15, wobei die eine oder die mehreren Kartuschen eine einzelne Kartusche umfassen, die optional eine einheitliche Struktur mit einer Vielzahl von Fluidkanälen und einer Vielzahl von Fluidkammern umfasst.

17. System nach einem der Ansprüche 12-16, wobei die Logik so konfiguriert ist, dass sie die genannten Operationen in einer Weise veranlasst, dass eines der Verfahren der Ansprüche 5 bis 9 durchgeführt wird.

18. System zur Vorbereitung einer Probe für einen Assay, wobei das System umfasst:
(a) eine Schnittstelle zum Aufnehmen und Bearbeiten von einer oder mehreren Kartuschen;
(b) eine Logik zur Veranlassung der Durchführung der folgenden Operationen in der einen oder mehreren Kartuschen: Aufnehmen einer DNA-haltigen Eingangsprobe;
Leiten eines ersten Teils der DNA aus der Eingangsprobe zu einer ersten PCR-Kammer der einen oder mehreren Kartuschen;
Leiten eines zweiten Teils der DNA aus der Eingangsprobe zu einer zweiten PCR-Kammer der einen oder mehreren Kartuschen;
Initiieren einer quantitativen PCR ("qPCR") am ersten Teil der DNA aus der Eingangsprobe in der ersten PCR-Kammer;
Initiieren einer Short-Tandem-Repeat("STR")-PCR am zweiten Teil der DNA aus der Eingangsprobe in der zweiten PCR-Kammer;
Detektieren eines Ausgangssignals von der ersten PCR-Kammer, wobei das Ausgangssignal Informationen über die DNA-Konzentration im ersten Teil der DNA aus der Eingangsprobe liefert; und
Abschließen der STR-PCR durch Durchführen einer festgelegten Anzahl von Amplifikationszyklen am zweiten Teil in der zweiten PCR-Kammer, wobei die festgelegte Anzahl von Amplifikationszyklen auf dem Ausgangssignal der ersten PCR-Kammer basiert.

19. Verfahren nach einem der Ansprüche 1-11 oder das System nach einem der Ansprüche 12-17, wobei das Leiten des zweiten Teils der DNA das Leiten des zweiten Teils der DNA aus der Eingangsprobe zu einer ersten PCR-Kammer und dann von der ersten PCR-Kammer zur zweiten PCR-Kammer umfasst.

20. Verfahren oder System zur Vorbereitung einer Probe für einen Assay nach einem der vorangegangenen Ansprüche, wobei der Assay die Kapillarelektrophorese von STR-Allelen ist.

## Revendications

1. Procédé de préparation d'un échantillon contenant de l'ADN pour un dosage, le procédé comprenant : dans une ou plusieurs cartouches, la réception d'un échantillon d'entrée comprenant de l'ADN ;
orientation d'une première partie de l'ADN de l'échantillon d'entrée vers une première chambre de PCR d'une première cartouche ;
orientation d'une deuxième partie de l'ADN de l'échantillon d'entrée vers une deuxième chambre de PCR de la première cartouche ou d'une deuxième cartouche ;
l'initiation d'un dosage PCR quantitatif (« PCRq ») sur la première partie dans la première chambre de PCR ;
avant de terminer le dosage PCRq dans la première chambre de PCR, l'initiation d'une PCR de courte répétition en tandem (Short Tandem Repeat, « STR ») sur la deuxième partie dans la deuxième chambre de PCR ;
la détection d'un signal de sortie de la première chambre de PCR, lequel signal de sortie fournit des informations sur la concentration d'ADN dans la première partie de l'ADN de l'échantillon d'entrée ; et
la réalisation de la PCR STR en effectuant un nombre prédéterminé de cycles d'amplification sur la deuxième partie dans la deuxième chambre de PCR, le nombre prédéterminé de cycles d'amplification étant basé sur le signal de sortie de la première chambre de PCR.

2. Procédé selon la revendication 1, l'échantillon d'entrée comprenant de l'ADN obtenu sur une scène de crime, facultativement l'ADN obtenu sur la scène de crime ayant été obtenu à partir d'une source sélectionnée dans le groupe composé d'os, de dents, de salive, de cheveux, de sang, de sperme, de peau et toute combinaison de ceux-ci.

3. Procédé selon l'une quelconque des revendications précédentes, la ou plusieurs cartouches comprenant une cartouche unique comprenant facultativement une structure unitaire composée d'une pluralité de canaux fluidiques et une pluralité de chambres fluidiques.

4. Procédé selon l'une quelconque des revendications précédentes, ni la première ni la deuxième partie de l'ADN de l'échantillon d'entrée n'étant diluée par ajout d'un liquide à la première ou à la deuxième partie de l'ADN de l'échantillon d'entrée avant l'initiation de la PCRq ou de la PCR STR.

5. Procédé selon l'une quelconque des revendications précédentes, dans lequel, après avoir réceptionné l'échantillon d'entrée, ni l'échantillon d'entrée ni la deuxième partie d'ADN de l'échantillon d'entrée ne sont quantifiés avant d'initier la PCR de STR.

6. Procédé selon l'une quelconque des revendications précédentes, dans lequel, après avoir commencé la PCRq mais avant de terminer la PCRq sur la première partie de l'ADN, la PCR STR est initiée en fournissant un ou plusieurs réactifs de PCR à la deuxième chambre de PCR et/ou en dirigeant la deuxième partie de l'ADN de l'échantillon d'entrée vers la deuxième chambre de PCR, facultativement l'initiation de la PCR STR commençant entre les cycles PCR cinq et quinze après le début de la PCRq.

7. Procédé selon la revendication 6, l'initiation de la PCR STR comprenant la fourniture d'un ou plusieurs réactifs de PCR à la deuxième chambre de PCR, et facultativement l'orientation de la première partie de l'ADN de l'échantillon d'entrée vers la première chambre de PCR et l'orientation de la deuxième partie de l'ADN de l'échantillon d'entrée dans la deuxième chambre de PCR se produisant à peu près en même temps.

8. Procédé selon l'une quelconque des revendications précédentes, comprenant en outre, avant de diriger la première partie de l'ADN de l'échantillon d'entrée vers une première chambre de PCR, la division de l'ADN de l'échantillon d'entrée dans la première partie et la deuxième partie d'ADN, et dans lequel la première partie de l'ADN de l'échantillon d'entrée a un volume s'élevant à environ vingt pour cent du volume de la deuxième partie d'ADN de l'échantillon d'entrée.

9. Procédé selon l'une quelconque des revendications précédentes, comprenant en outre l'extraction de l'ADN de l'échantillon d'entrée avant d'initier la PCRq sur la première partie de l'ADN de l'échantillon d'entrée, facultativement l'extraction de l'ADN comprenant la mise en contact de l'échantillon d'entrée avec des billes de capture d'ADN.

10. Procédé selon l'une quelconque des revendications précédentes, la deuxième chambre de PCR comprenant un matériau absorbant qui retient la deuxième partie de l'ADN de l'échantillon d'entrée pendant la PCR STR.

11. Procédé selon l'une quelconque des revendications précédentes, la PCRq et la PCR STR étant effectuées à l'aide d'un thermocycleur unique, et pendant au moins une partie de la PCRq effectuée en même temps qu'au moins une partie de la PCR STR, la PCRq et la PCR STR étant soumises au même profil thermique ; ou la PCRq et la PCR STR étant effectuées à l'aide de thermocycleurs distincts.

12. Système de préparation d'un échantillon pour un dosage, le système comprenant :
(a) une interface pour la réception et l'exploitation d'une ou plusieurs cartouches ;
(b) une logique amenant les opérations suivantes à être effectuées dans l'une ou plusieurs cartouches : la réception d'un échantillon d'entrée comprenant de l'ADN ;
orientation d'une première partie de l'ADN de l'échantillon d'entrée vers une première chambre de PCR d'une première cartouche ;
orientation d'une deuxième partie de l'ADN de l'échantillon d'entrée vers une deuxième chambre de PCR de la première cartouche ou d'une deuxième cartouche ;
initiation d'une PCR quantitative (« PCRq ») sur la première partie d'e l'ADN de l'échantillons d'entrée dans la première chambre de PCR ;
avant de terminer la PCRq dans la première chambre de PCR, initiation d'une PCR de courte répétition en tandem (Short Tandem Repeat, « STR ») sur la deuxième partie de l'ADN dans la deuxième chambre de PCR ;
la détection d'un signal de sortie de la première chambre de PCR, lequel signal de sortie fournit des informations sur la concentration d'ADN dans la première partie de l'ADN de l'échantillon d'entrée ; et
réalisation de la PCR STR en effectuant un nombre prédéterminé de cycles d'amplification sur la deuxième partie dans la deuxième chambre de PCR, le nombre prédéterminé de cycles d'amplification étant basé sur le signal de sortie de la première chambre de PCR.
Et facultativement, comprenant en outre un ou plusieurs actionneurs pour contrôler de façon dynamique le mouvement des fluides dans les passages fluidiques de la ou de plusieurs cartouches.

13. Système selon la revendication 12, comprenant en outre un thermocycleur configuré pour provoquer la PCRq dans la première chambre de PCR ou comprenant en outre, un thermocycleur configuré pour (i) provoquer la PCRq dans la première chambre de PCR et (ii) provoquer la PCR STR dans la deuxième chambre de PCR.

14. Système selon l'une quelconque des revendications 12 à 13, comprenant en outre :
(a) un capillaire contenant un milieu de séparation et ayant des extrémités d'entrée et distale ;
(b) une entrée en communication avec l'entrée du capillaire et configurée pour recevoir des échantillons contenant des fragments d'ADN ayant une pluralité de tailles différentes ;
(c) une région d'interrogation configurée pour détecter des fragments d'ADN se déplaçant dans le capillaire ; et
(d) une source d'alimentation configurée pour appliquer une tension entre les extrémités d'entrée et distale du capillaire.

15. Système selon l'une quelconque des revendications 12 à 14, comprenant en outre un châssis renfermant l'interface et au moins une partie de la logique.

16. Système selon l'une quelconque des revendications 12 à 15, la ou plusieurs cartouches comprenant une seule cartouche comprenant facultativement une structure unitaire comprenant une pluralité de canaux fluidiques et une pluralité de chambres fluidiques.

17. Système selon l'une quelconque des revendications 12 à 16, la logique étant configurée pour provoquer les opérations décrites d'une manière telle que l'un quelconque des procédés selon les revendications 5 à 9 soit effectué.

18. Système de préparation d'un échantillon pour un dosage, le système comprenant :
(a) une interface pour la réception et l'exploitation d'une ou plusieurs cartouches ;
(b) une logique amenant les opérations suivantes à être effectuées dans l'une ou plusieurs cartouches : la réception d'un échantillon d'entrée comprenant de l'ADN ;
l'orientation d'une première partie d'ADN de l'échantillon d'entrée vers une première chambre de PCR d'une première cartouche ;
l'orientation d'une deuxième partie d'ADN de l'échantillon d'entrée vers une deuxième chambre de PCR de la ou de plusieurs cartouches ;
initiation d'une PCR quantitative (« PCRq ») sur la première partie d'e l'ADN de l'échantillons d'entrée dans la première chambre de PCR ;
l'initiation d'une PCR de courte répétition en tandem (Short Tandem Repeat, « STR ») sur la deuxième partie de l'ADN de l'échantillon d'entrée dans la deuxième chambre PCR ;
la détection d'un signal de sortie de la première chambre de PCR, lequel signal de sortie fournit des informations sur la concentration d'ADN dans la première partie de l'ADN de l'échantillon d'entrée ; et
la réalisation de la PCR STR en effectuant un nombre prédéterminé de cycles d'amplification sur la deuxième partie dans la deuxième chambre de PCR, le nombre prédéterminé de cycles d'amplification étant basé sur le signal de sortie de la première chambre de PCR.

19. Procédé selon l'une quelconque des revendications 1 à 11 ou système selon l'une quelconque des revendications 12 à 17, l'orientation de la deuxième partie de l'ADN comprenant l'orientation de la deuxième partie de l'ADN de l'échantillon d'entrée vers une première chambre de PCR, puis de la la première chambre de PCR vers la deuxième chambre de PCR.

20. Procédé ou système pour préparer un échantillon pour un dosage selon l'une quelconque des revendications précédentes, le dosage étant une électrophorèse capillaire d'allèles STR.
